# EUROPEAN PATENT APPLICATION

(11) **EP 3 957 720 A1**
(43) Date of publication of application: **23.02.2022**
(21) Application number: 20192262.2
(22) Date of filing: 21.08.2020
(51) Int. Cl.: C12N 5/0783, A61P 31/12, A61P 35/00, C12N 9/22, C12N 15/85

(54) **IMMUNOSUPPRESSANT-RESISTANT T-CELLS FOR ADOPTIVE IMMUNOTHERAPY**

(71) Applicant: Charité - Universitätsmedizin Berlin, 10117 Berlin (DE)
(72) Inventor: Amini, Leila, 13353 Berlin (DE); Schmück-Henneresse, Michael, 10247 Berlin (DE); Reinke, Petra, 10117 Berlin (DE); Volk, Hans-Dieter, 10117 Berlin (DE); Wagner, Dimitrios Laurin, 13347 Berlin (DE)
(74) Representative: Moré, Solveig Helga

(57) **Abstract**

The present invention provides to the field of cell therapy, in particular, to adoptive T cell or NK cell therapy. It provides a human immunophilin knockout cell that is a T cell or NK cell, wherein the immunophilin is FKBP12 or cyclophilin A, or a cell population of such cells. The cell is resistant to the action of the immunosuppressant Tacrolimus and/or cyclosporine A. It is obtainable by CRISPR/Cas, e.g., CRISPR/Cas9-mediated knockout of the immunophilin or a derivate technology. The cell can be, e.g., a virus-specific cell, e.g., specific for an antigen from any of CMV, EBV, BKV, HPV, ADV, influenza virus, or SARS-CoV-2. It may also be a regulatory T cell. The invention also provides a method for preparing the human immunophilin knockout T cell or NK cell of the invention, pharmaceutical compositions or kits comprising said cells, pharmaceutical compositions or kits for use in in treatment or prevention of an infection with a virus or another pathogen or for use in treatment of cancer, or for use in balancing an unwanted immune response, e.g., in the context of a condition selected from the group comprising autoimmunity, allergy, a transplantation and bystander activation. Methods of treatment are also disclosed.

SEQUENCE LISTING

<110> Charité - Universitätsmedizin Berlin

<120> Immunosuppressant-resistant T-cells for adoptive immunotherapy

<130> 12761 P 6821 EP

<160> 27

<170> BiSSAP 1.3.6

<210> 1
<211> 20
<212> DNA
<213> Homo sapiens

<220>
<223> Target sequence of sgRNA #2

<400> 1
gggcgcacct tccccaagcg                                                20

<210> 2
<211> 20
<212> DNA
<213> Homo sapiens

<220>
<223> Target sequence of sgRNA #1

<400> 2
gcactacacc ggtgagtcgg                                                20

<210> 3
<211> 20
<212> DNA
<213> Homo sapiens

<220>
<223> Target sequence of sgRNA #3

<400> 3
cggtgagtag tggcgcgcgg                                                20

<210> 4
<211> 20
<212> DNA
<213> Homo sapiens

<220>
<223> Target sequence of sgRNA #4

<400> 4
cgtggtataa aaggggcggg                                                20

<210> 5
<211> 20
<212> DNA
<213> Homo sapiens

<220>
<223> Target sequence of sgRNA #5

<400> 5
cggcggtggc gtctgcaaaa                                                    20

<210> 6
<211> 20
<212> DNA
<213> Homo sapiens

<220>
<223> Target sequence of sgRNA #6

<400> 6
cgccgcccgc ccgacctcaa                                                    20

<210> 7
<211> 20
<212> DNA
<213> Homo sapiens

<220>
<223> Target sequence of sgRNA #7

<400> 7
gccgcgtctc ctttgaggtc                                                    20

<210> 8
<211> 24
<212> DNA
<213> Artificial Sequence

<220>
<223> FKBP12 amplification primer forward (sgRNA #1&2)

<400> 8
tctgacgggt cagataacac ctag                                               24

<210> 9
<211> 20
<212> DNA
<213> Artificial Sequence

<220>
<223> FKBP12 amplification primer reverse (sgRNA #1&2)

<400> 9

tcttccggag gcctgggttt                                                    20

<210> 10
<211> 20
<212> DNA
<213> Artificial Sequence

<220>
<223> FKBP12 amplification primer forward (sgRNA #3)

<400> 10
acagctgtat ccggaggcct                                                    20

<210> 11
<211> 20
<212> DNA
<213> Artificial Sequence

<220>
<223> FKBP12 amplification primer reverse (sgRNA #3)

<400> 11
tcacagccgc cgattcagac                                                    20

<210> 12
<211> 21
<212> DNA
<213> Artificial Sequence

<220>
<223> PPIA amplification primer (forward)

<400> 12
actcgcggac ctcccaaaat g                                                  21

<210> 13
<211> 20
<212> DNA
<213> Artificial Sequence

<220>
<223> PPIA amplification primer (reverse)

<400> 13
acgcgcctca tcgcttgaca                                                    20

<210> 14
<211> 20
<212> RNA
<213> Artificial Sequence

<220>
<223> sgRNA #2 - spacer sequence of guide RNA

<400> 14
gggcgcaccu uccccaagcg                                                   20


<210> 15
<211> 20
<212> RNA
<213> Artificial Sequence


<220>
<223> sgRNA #1 - spacer sequence of guide RNA

<400> 15
ggacuacacc ggugagucgg                                                   20


<210> 16
<211> 20
<212> RNA
<213> Artificial Sequence


<220>
<223> sgRNA #3 - spacer sequence of guide RNA

<400> 16
gggugaguag uggcgcgcgg                                                   20


<210> 17
<211> 96
<212> RNA
<213> Artificial Sequence


<220>
<223> sgRNA #2 - full sequence (with standard scaffold sequence for
      SpCas9)

<400> 17
gggcgcaccu uccccaagcg guuuuagagc uagaaauagc aaguuaaaau aaggcuaguc      60

cguuaucaac uugaaaaagu ggcaccgagu cggugc                                 96


<210> 18
<211> 96
<212> RNA
<213> Artificial Sequence


<220>
<223> sgRNA #1 - full sequence (with standard scaffold sequence for
      SpCas9)

<400> 18
gcacuacacc ggugagucgg guuuuagagc uagaaauagc aaguuaaaau aaggcuaguc      60

cguuaucaac uugaaaaagu ggcaccgagu cggugc                                 96

<210> 19
<211> 96
<212> RNA
<213> Artificial Sequence

<220>
<223> sgRNA #3 - full sequence (with standard scaffold sequence for
      SpCas9)

<400> 19
cggugaguag uggcgcgcgg guuuuagagc uagaaauagc aaguuaaaau aaggcuaguc      60

cguuaucaac uugaaaaagu ggcaccgagu cggugc                                96

<210> 20
<211> 20
<212> RNA
<213> Artificial Sequence

<220>
<223> sgRNA #4 - spacer sequence of guide RNA

<400> 20
ggugguauaa aaggggcggg                                                  20

<210> 21
<211> 20
<212> RNA
<213> Artificial Sequence

<220>
<223> sgRNA #5 - spacer sequence of guide RNA

<400> 21
gggcgguggc gucugcaaaa                                                  20

<210> 22
<211> 20
<212> RNA
<213> Artificial Sequence

<220>
<223> sgRNA #6 - spacer sequence of guide RNA

<400> 22
ggccgcccgc ccgaccucaa                                                  20

<210> 23
<211> 20
<212> RNA
<213> Artificial Sequence

<220>
<223> sgRNA #7 - spacer sequence of guide RNA

<400> 23
gccgcgucuc cuuugagguc                                                   20


<210> 24
<211> 96
<212> RNA
<213> Artificial Sequence


<220>
<223> sgRNA #4 - full sequence (with standard scaffold sequence for
      SpCas9)

<400> 24
cgugguauaa aaggggcggg guuuuagagc uagaaauagc aaguuaaaau aaggcuaguc      60

cguuaucaac uugaaaaagu ggcaccgagu cggugc                                96


<210> 25
<211> 96
<212> RNA
<213> Artificial Sequence


<220>
<223> sgRNA #5 - full sequence (with standard scaffold sequence for
      SpCas9)

<400> 25
cggcgguggc gucugcaaaa guuuuagagc uagaaauagc aaguuaaaau aaggcuaguc      60

cguuaucaac uugaaaaagu ggcaccgagu cggugc                                96


<210> 26
<211> 96
<212> RNA
<213> Artificial Sequence


<220>
<223> sgRNA #6 - full sequence (with standard scaffold sequence for
      SpCas9)

<400> 26
cgccgcccgc ccgaccucaa guuuuagagc uagaaauagc aaguuaaaau aaggcuaguc      60

cguuaucaac uugaaaaagu ggcaccgagu cggugc                                96


<210> 27
<211> 96
<212> RNA
<213> Artificial Sequence


<220>
<223> sgRNA #7 - full sequence (with standard scaffold sequence for
      SpCas9)

<400> 27
gccgcgucuc cuuugagguc guuuuagagc uagaaauagc aaguuaaaau aaggcuaguc 60

cguuaucaac uugaaaaagu ggcaccgagu cggugc 96

## Description

The present invention relates to the field of cell therapy, in particular, to adoptive T cell or NK cell therapy. It provides a concept of targeted reshaping a pathogenic immune imbalance by a combination of immunosuppression, in particular calcineurin inhibitor alone or in combination with corticosteroids, with a highly specialized human cell product with immunophilin knockout alone or in combination with steroid receptor knockout. The invention thus provides a T cell or NK cell, wherein the immunophilin is FK506-binding protein 12 (FKBP12) or cyclophilin A, or a cell subset or population of such cells. The gene encoding cyclophilin A is commonly designated PPIA, peptidylprolyl isomerase A. The cell is resistant to the action of the immunosuppressant Tacrolimus and/or cyclosporine A. This immunophilin knockout can be combined with knockout of the nuclear receptor subfamily 3 group C member 1 (NR3C1; the gene encoding for the glucocorticoid receptor). The cell is then resistant to the immunosuppressants Tacrolimus and/or cyclosporine A as well as, optionally, the immunosuppressive corticosteroids (cortisol, prednisolone, methylprednisolon, dexamethasone). It is obtainable by gene editing, in particular, Clustered Regularly Interspaced Short Palindromic Repeats (CRISPR)-Cas-based knockout of the immunophilin, e.g., by CRISPR-Cas9. The invention thus provides a vector-free CRISPR-Cas-ribonucleoprotein-based good manufacturing practice (GMP)-compliant protocol, which efficiently targets and prevents the production of functional adaptor protein FKBP12 required for the immunosuppressive function of Tacrolimus or of cyclophilin A required for the immunosuppressive function of cyclosporine A and, optionally, of the glucocorticoid receptor required for the lymphotoxic function of glucocorticoids. The cell can be, e.g., a virus-specific cell to restore protection to viral infections, e.g., specific for an antigen from any of CMV, EBV, BKV, HPV, ADV, influenza virus, or SARS-CoV-2. It could be also a tumor antigen-specific effector cell, targeting cancer cells. It may also be a regulatory T cell, a specialized cell type to prevent overwhelming immunity/inflammation. The invention further provides a method for preparing the human immunophilin knockout T cell or NK cell of the invention, pharmaceutical compositions or kits comprising said cells, pharmaceutical compositions or kits for use in in treatment or prevention of an infection with a virus or another pathogen or for use in treatment of cancer, or for use in balancing an unwanted immune response, e.g., in the context of a condition selected from the group comprising autoimmunity, allergy, a transplantation and bystander activation. Methods of treatment are also disclosed.

Viral diseases are often associated with unwanted immune responses, e.g. allogenic organ rejection in solid organ transplant (SOT) recipients or acute T cell immunopathology caused by bystander T-cell activation during acute viral infections. Viral infections, such as cytomegalovirus (CMV), Epstein-Barr-Virus and BK-Virus, remain a major problem for morbidity and mortality in chronically immunocompromised patients. An exemplary case is cytomegalovirus (CMV) disease, a life-threatening complication occurring due to the immunosuppressive medication required for prevention of organ rejection in solid organ transplant (SOT) recipients. CMV disease causes direct and indirect morbidities including rejection of the transplanted organ¹ or chronic allograft nephropathy in kidney transplantation (KTx)². Classical antiviral medication is used as prophylaxis, pre-emptive or rescue therapy, though careful dosing is crucial to prevent toxicities and resistance³. Nevertheless, high frequencies of late-onset CMV disease have been observed with increased mortality among CMV-infected KTx recipients compared to uninfected recipients⁴. The T-cell-mediated anti-CMV response has proven to be a suitable predictive and stratification marker for CMV disease outcome^{5, 6}. Thus, regeneration of the endogenous T-cell response by adoptive anti-viral T-cell therapy may diminish CMV-associated morbidities and mortality after transplantation.

Antiviral T-cell therapy is an established method to reconstitute effective immunity in hematopoietic stem cell transplant (HSCT) recipients⁷⁻¹². The inventors have previously confirmed that antiviral T-cell products (TCPs) can be successfully generated from immunosuppressed solid organ transplant recipients^{13, 14}. Treatment of CMV-disease using adoptive T-cell transfer to solid organ transplant recipients has been reported in a few studies¹⁵⁻¹⁷. However, despite the initial efficient reduction of symptoms and viral load^{18, 19}, limited persistence and longevity of the TCP represent major challenges for successful adoptive cell therapy for CMV-disease. Similarly, relapses in Epstein-Barr-Virus load have been documented in TCP-treated SOT patients²⁰.

One reason for limited long-term efficacy of antiviral T-cell therapy may be the ongoing immunosuppression required to prevent organ rejection in solid organ transplant recipients. Conventional immunosuppression for KTx (and other solid organ transplant patients) may comprise at least one of three classes of immunosuppressants: Calcineurin inhibitors (CNIs, e.g. Tacrolimus [FK506] and Cyclosporine A [CsA]) potently reduce T-cell activation, maturation and cytokine secretion²³. Glucocorticoids blunt cytokine production by T-cells²⁴ as well as provoking lymphocyte apoptosis²⁵. The proliferation inhibitor Mycophenolic acid (MPA), which also inhibits pro-inflammatory cytokine production²¹, induces lymphocyte apoptosis and decreases homing of T-cells²². Preformed allo-antigen-reactive effector T-cells are particularly difficult to control in transplant recipients.

Calcineurin inhibitors are currently the most effective treatment for the sustained suppression of these allo-antigen-reactive cells. Therefore, solutions are required to maintain control of allograft reactive T-cells whilst overcoming suppression of protective antiviral T-cell responses. To circumvent the effects of Tacrolimus on adoptively transferred virus-specific TCPs, Tacrolimus-insensitive forms of calcineurin^{26, 27} have been introduced into cells using retroviruses. Another method to induce Tacrolimus insensitivity is the use of a retrovirally integrated siRNA expression cassette to knockdown the immunophilin FKBP12²⁸, the adaptor protein needed for the immunosuppressive function of Tacrolimus in T-cells²⁹. However, retroviruses integrate into the genome randomly, therefore posing a possible safety risk due to the potential for gene disruption or dysregulation. Furthermore, the mutated proteins introduced are putatively immunogenic. Moreover, RNA interference is often incomplete, thus partial susceptibility to Tacrolimus may be retained. Neither of these approaches has yet reached clinical application.

Severe or chronic viral diseases also cause T-cell immunopathology due to bystander activation, e.g., of highly differentiated memory T-cells independent of their T-cell receptor specificity, for example via IL-15, especially in the elderly population possessing highly differentiated T-cells.

In light of this, the inventors have addressed the problem of providing an improved T cell or NK cell that may be used in a cell product for adoptive transfer to a patient, e.g., in a patient wherein an undesired immune response is inhibited by a calcineurin inhibitor. The aim is to allow specific T cells to react to their targets adequately, at the same time as the patient receives immunosuppressant chemotherapy to limit a pathological immune response.

The present invention provides a human immunophilin knockout cell that is a T cell or NK cell, wherein the immunophilin is FKBP12 or Cyclophilin A. Cyclophilin A is also designated peptidylprolyl isomerase A or PPIA. The gene is commonly designated PPIA. The terms are used interchangeably herein. In the context of the invention, a knockout cell does not comprise a functional allele of the knocked out immunophilin gene. This renders the cell resistant to an immunosuppressant capable of interacting with the said immunophilin, i.e., a calcineurin inhibitor. In particular, if the immunophilin is FKBP-12, the cell is resistant to Tacrolimus. If the immunophilin is cyclophilin A, the cell is resistant to Cyclosporine A (CsA).

The invention thus provides a GMP-compliant, vector-free, gene-editing approach targeting the FKBP12 or PPIA gene to make CMV-specific Tacrolimus- or cyclosporin-resistant T-cells. Efficient knockout, or gene editing, is achieved by transfection of ribonucleoprotein complexes (RNP) comprising CRISPR-associated protein (Cas), e.g., Cas9 and respective single sgRNA (sgRNA)³⁰. Alternatively, other CRISPR-associated proteins, such as Cas12a, or related technologies such as base editor proteins or prime editing technology may be used instead of the Cas9 protein. The inventors implemented a RNP nucleofection step, e.g., in their existing clinical protocol for the generation of antiviral TCPs¹⁹. For safety, the protocol comprises an initial sorting step, e.g, to solely expand T-cells of a certain specificity (e.g. virus-specific T-cells), activated upon exposure to the respective (viral) antigens²⁷. Functionally, immunophilin^{k.o.} antiviral T-cells, e.g., FKBP12^{k.o.} cells showed superior cytokine production and activation in the presence of the calcineurin inhibitor compared to unedited antiviral T-cells. In the case of FKBP-12^{k.o.}, these properties were inhibited in the presence of an alternative calcineurin inhibitor, CsA, thereby demonstrating the specificity of pathway inhibition and providing an optional safety switch to control potential undesired effects of gene-edited FKBP12^{k.o.} T-cells in patients. By combining multiple highly refined technologies, the inventors have developed a unique approach for the efficient and safe generation of clinical-grade calcineurin inhibitor -resistant TCPs or NK cell products suitable for adoptive T-cell therapy or adoptive NK cell therapy.

The invention can be used in two scenarios to reshape unwanted immune imbalance:
i) patients suffering from strong unwanted immune response (e.g. alloimmunity in organ or hematopoetic stem cell transplantation, or uncontrolled hyperimmunity as in severe atopic eczema) who routinely receive calcineurin inhibitors (CyA or tacrolimus) which effectively suppress the unwanted immune response but also the protective anti-viral T-cell response. Consequently, patients are at high risk of infectious complications and virus-related tumors, frequently associated with need for reducing immunosuppression resulting in relapse of the underlying disease. The use of specific adoptive antiviral T/NK cell therapy can improve the pathogen control, but its efficacy is limited due to sensitivity of those cells to calcineurin inhibitors as well. The invention provides calcineurin inhibitor resistant (or combination with steroid-resistant) anti-viral cells that can effectively protect even in presence of basal immunosuppression, thus overcoming the need for weaning of calcineurin inhibitors in those patients. Moreover, unwanted immune reactivity is limited by self-regulation *via* regulatory T cells. Unfortunately, they are also sensitive to calcineurin inhibitors, meaning that both bad and good players are suppressed, preventing endogenous reshaping of unwanted immune responses. The invention provides a tool, calcineurin inhibitor-resistant (or combination with steroid-resistant) Treg, that can reshape unwanted immune response in presence of immunosuppression to allow long-lasting weaning of the latter.
ii) The pathogenicity and mortality associated with viral infections in non-immunosuppressed patients, such as influenza, SARS-CoV2, hepatitis B/C etc., is often more related to unspecific immune overactivation (bystander activation) than to the pathogen itself. Although immunosuppression with calcineurin inhibitors (alone or in combination with steroids) or the application of regulatory T cells would be useful, it is commonly not used because of the undesired inhibition of protective antiviral response resulting in an unfavourable risk/benefit analysis. The inventions provides a concept to combine useful immunosuppression in particular by calcineurin inhibitor alone or in combination with steroids by adoptive transfer of calcineurin inhibitor-resistant (optionally, in combination with steroid resistant) protective anti-viral cells and/or calcineurin inhibitor-resistant (optionally, in combination with steroid resistance) regulatory T cells to reshape unwanted bystander reactions without affecting protective immunity.

In a preferred embodiment, the cell is a T cell. Adoptive T cell therapy is more commonly used than NK cell therapy, for example, adoptive T cell therapy may be used in treatment of cancer or an infection, e.g., a virus infection. The T cell may be a CD4+ T cell. However, in particular in the context of virus-specific T cells, the T cell may be a CD8+ T cell.

In the context of the invention, "a" is to be understood as "at least one", unless explicitly mentioned otherwise. Thus, the method also relates to human immunophilin knockout cells that are a T cells or NK cells, wherein the immunophilin is FKBP12 or Cyclophilin A. These cells may also, in a preferred embodiment, comprise a mixture of CD4+ and CD8+ T cells. Preferably, both CD4+ and CD8+ T cells are specific for the same antigen, or the same antigens. The antigen or antigens may be derived from one or multiple pathogens, e.g., from one virus, or from multiple viruses or a tumor-antigen.

The cell may be a T cell specific for a virus, another pathogen or a cancer antigen. For example, the T cell may be a virus-specific T cell, wherein the virus is selected from the group comprising CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-2. The T cells may also be multi-virus specific T cells, e.g., T cells activated and selected by addition of antigens from multiple viruses.

Alternatively, the cell is a T cell specific for a cancer antigen, e.g., a tumor-specific antigen, a differentiation antigen, a viral antigen, a cancer-testis antigen. Typical cancer antigens are CD19, CD38, MAGE-A1, CTAG1B, AFP, CEA, CA-125, ETA, tyrosinase, or NY-ESO-1.

In another embodiment of the invention, the cell is a regulatory T cell, preferably, a CD4+CD25+ regulatory T cell, optionally, a CD4+CD25+ CD127⁻ regulatory T cell. It may be a regulatory T cell product consisting of polyclonal repertoire or specific for a defined antigen, e.g., a certain allogenic MHC molecule, autoantigen, or a virus such as MERS, SARS-CoV1 and SARS-CoV-2.

Alternatively, the cell may also be an NKT cell, an NK cell or a γδ T cell. NK cells are particularly useful for cancer indications (solid, haematological), in viral infection, such as CMV or EBV, bacterial infections, in macrophage activation syndrome or similar. NKT cells may be used for cancer indications (solid, haematological), in viral infection, such as CMV or EBV, in macrophage activation syndrome or similar. γδ T cells may be used for cancer indications (solid, haematological), in viral infection, such as CMV or EBV, bacterial infections.

The cell of the invention, e.g., the T cell of the invention, may comprise at least one, e.g., two or three further genetic modifications.

For example, the cell may comprise a) a knockout of both immunophilin genes as defined in claim 1, so that the genes encoding FKBP12 and cyclophilin A are knocked out. Preferably, the glucocorticoid receptor gene is functional. In this case, steroid therapy provides a safety switch in case there are any issues with the transgenic T cells of the invention, e.g., any pathogenicity caused by these cells.

Alternatively, the cell may comprise b) a knockout of the glucocorticoid receptor gene, and a knockout of one of the immunophilin genes encoding either FKBP12 or Cyclophilin A, whereas the second immunophilin gene is functional. An immunosuppressant capable of interacting with the second immunophilin can thus be used as a safety switch in case there are any issues with the transgenic T cells of the invention, e.g., any pathogenicity caused by these cells. If there is more than one genetic modification in the cell relating to resistance to immunosuppressants, this embodiment is preferred, as the safety switch is robust and can easily be used in the clinic.

In a further embodiment, the cell may comprise c) a knockout of both immunophilin genes encoding FKBP12 and Cyclophilin A, so that the genes encoding FKBP12 and Cyclophilin A are knocked out, and of the glucocorticoid receptor gene.

The invention also provides a cell population comprising cells of the invention, preferably, exclusively cells of the invention. Preferably, unless the T cell comprises a transgenic TCR or a chimeric antigen receptor, the cell population is a polyclonal population of cells. It is however preferably a T cell population comprising, to a large extent e.g., at least 70%, at least 80%, at least 90%, at least 95% or at least 99% or 100% T cells that are homogenous with regard to specific characteristics, e.g., regulatory T cells, T cells specific to one or more antigens from a defined pathogen, e.g., virus, T cells specific to a defined common antigen (but not necessarily to a common epitope), NKT cells or γδ T cells.

The cells of the invention are obtainable by gene editing. The preferred gene editing method of the invention comprises CRISPR/Cas-mediated gene editing. This employs regular double-strand cutting nuclease enzymes such as, but not only, Cas9 (derived from *Streptococcus pyogenes* or functional mutants thereof) or Cas12a (derived from *Acidaminococcus sp.* or functional mutants thereof). Other Cas enzymes, e.g., *Staphylococcus aureus* Cas9, *Streptococcus thermophiles* Cas9, or LbCas12a can also be used.

Thus, the cell population of the invention preferably comprises cells with InDel mutations in the genes (i.e. in both genes) encoding the immunophilin. For example, at least 10% of the cells of the population comprise said InDel mutations, optionally, at least 20%, preferably, at least 50%, e.g., at least 70%, at least 90% or 100%. InDel mutations are characteristic mutations or deletions introduced by Cas, e.g., Cas9, around the cut site, which is dependent on the sgRNA⁴³. Preferably, in case of a FKBP12^{k.o.}, the InDel mutations are obtainable by CRISPR/Cas (e.g., Cas9)-mediated gene editing with a sgRNA targeting any of NO: 1, 2 or 3, preferably, SEQ ID NO: 1. Preferably, in case of a Cyclophilin^{k.o.}, the InDel mutations are obtainable by CRISPR/Cas (e.g., Cas9)-mediated gene editing with a sgRNA targeting SEQ ID NO: 4, 5, 6 or 7, preferably, 5 or 6.

Cas enzymes can also be genetically modified Cas enzymes. A functional mutant of a wildtype enzyme may, e.g., have at least 70%, at least 80% or at least 90% sequence identity to the wildtype enzyme and retains the ability to mediate gene editing, in particular, editing of the targets described herein. They can also be Cas derivative enzymes, including e.g. high fidelity enzymes (Vakulskas et al., 2018. Nat Medicine 24: 1216-1224), base editing enzymes (Komor et al. 2016. Nature 533:420-424; Rees and Liu 2019. Nat Rev Genet. 19(12):770-788; Walton et al., 2020. Science 368(6488): 290-296) or prime editors (Anzalone et al., 2019. Nature 576:149-157). Gene editing can also be performed with other programmable nucleases such as TALEN or Zinc Finger nucleases, including respective derivative enzymes. However, Cas enzymes are preferred.

CRISP/Cas-mediated gene editing in the context of the invention is understood to comprise derivative technologies such as base editing or prime editing. Accordingly, if the cells of the invention are generated using CRISPR/Cas derivative technologies such as base editor proteins or prime editing, the genetic intervention can also be used to insert stop codons in the reading frame, e.g., the early reading frame, or disrupt splice acceptor/donor sites, to prevent generation of functional immunophilin protein. The cells of the invention then comprise a stop codon in the reading frame of the knocked out gene, and/or a disrupted splice acceptor/donor site.

Preferably, the cell population of the invention is obtainable from Cas (e.g., Cas9)-mediated gene editing of the immunophilin genes of a respective cell population obtained from a human subject, e.g., with a sgRNA targeting any of SEQ ID NO: 1, 2 or 3, preferably, SEQ ID NO: 1 (for knockout of FKBP12).

If cyclophilin A is knocked out, the cell population of the invention is preferably obtainable from Cas (e.g., Cas9)-mediated gene editing of the immunophilin genes of a respective cell population obtained from a human subject, e.g., with a sgRNA targeting any of SEQ ID NO: 4, 5, 6 and/or 7, preferably, a combination thereof.

The InDel mutations can be analysed, e.g., as described in the examples below under "efficiency analysis" for the respective sgRNAs.

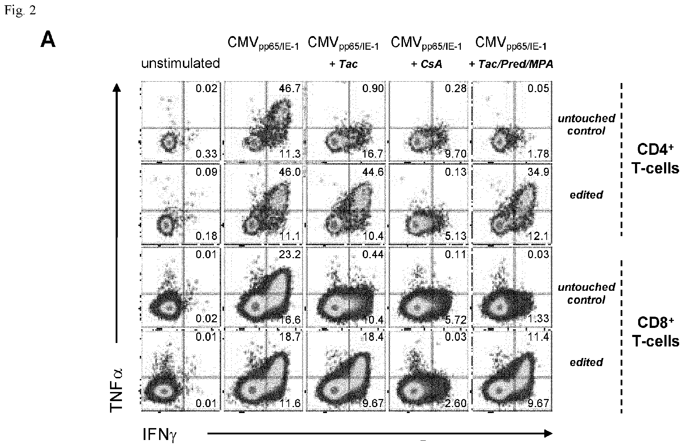

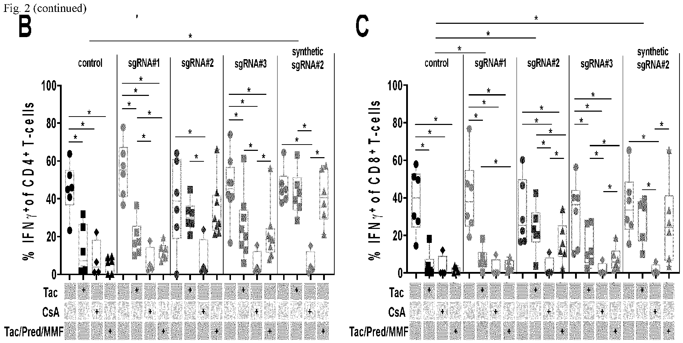

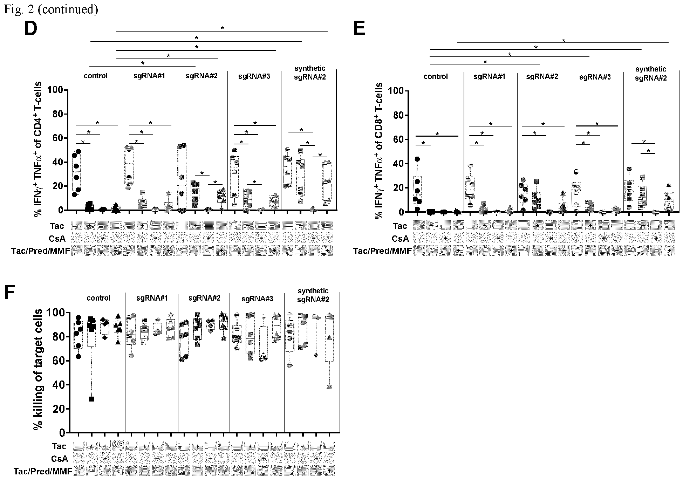

As a consequence of the knockout, the cells of the population, e.g., at least 10% of the cells of the population are resistant to the action of an immunosuppressant agent capable of interacting with the immunophilin, optionally, at least 20%, preferably, at least 50%, e.g., 70%, at least 90%, at least 99% or most preferably, 100%. In particular, resistant to the action of an immunosuppressant agent capable of interacting with the immunophilin means, that, in the presence of said immunosuppressant, the cells produce approximately the same amount of cytokines, e.g., IFNy for antigen-specific T cells (or, for regulatory T cells, IL-10) under the conditions exemplified in the experiments underlying Fig. 2 b and c, using the relevant antigen).

The invention also provides a method for preparing a human immunophilin knockout T cell or NK cell of the invention or a T cell or NK cell population of the invention, comprising
a) stimulating T cells or NK cells obtained from a subject,
b) isolating stimulated T cells or NK cells based on expression of a marker to obtain a composition of selected T cells or NK cells, and
c) gene editing the cells of said composition to knock out the gene encoding the immunophilin, e.g., introducing a ribonucleoprotein complex comprising Cas and a guide RNA, preferably, a sgRNA, targeting the gene encoding the immunophilin into the T cells of said composition.

The method may further comprise d) selecting immunophilin knockout cells by culturing the cells in the presence of an immunosuppressant agent capable of interacting with the immunophilin. Said step is however not required, as efficacy of knockout is commonly very high (>75%).

Preferably, the cell is a T cell. It is also preferred that the method is carried out with a plurality of T cells to obtain a cell population of the invention, e.g., a T cell population of the invention.

The cell can be obtained from a sample isolated from the subject. The subject preferably is a human subject. The cell, i.e., the T cell or NK cell, may be from the peripheral blood of the subject, e.g., from peripheral blood mononuclear cells (PBMC), e.g., isolated by density gradient or leukopheresis. Alternatively, the cells may be tumor-infiltrating lymphocytes, or they may be obtained from another tissue sample, or may be generated from iPSC.

The subject may be a patient, e.g., a patient requiring immunosuppression, for example because of a transplantation or another unwanted immune response, such as autoimmunity, allergy or unspecific immunopathology. The patient may already be immunosuppressed, or immunosuppression may be intended in the future, e.g., after the sample is taken. However, the subject may also be a healthy subject, wherein knockout cells of the invention may be produced for pharmaceutical use in a different patient, wherein said patient and said subject share at least one MHC allele, preferably, at least two, at least three, at least four or all MHC alleles. If the transferred cells are CD4+ cells, at least one MHC II allele should be shared, and/or if the transferred cells are CD8+ cells, at least one MHC I allele should be shared.

If the human immunophilin knockout cell of the invention is an antigen-specific T cell, in particular, a T cell specific for a virus, another pathogen or a cancer antigen, in step a), the T cells are stimulated with the antigen, e.g., a virus-derived antigen, another pathogen-derived antigen or a cancer antigen. The antigen can be part of an antigen mixture, e.g., a peptide mixture, a protein, a peptide- or protein pulsed antigen-presenting cell, or an antigen-presenting cell expressing said antigen. A single epitope may be used, but it is advantageous to use several, if not all epitopes of an antigen to stimulate a variety of antigen-specific T cells. Further, in some embodiments stimulations with multiple antigens of the same or distinct pathogens, e.g., multiple viruses, can be performed to generate highly selected, but multi-specific T cell lines. Stimulation can be for a suitable period of time to allow for expression of an activation marker, e.g., for 3-48 hours, for 4-24 hours or for 6 hours to overnight stimulation, e.g., about 12 hours. About, if used in the context of the present invention, preferably means +/- 10%.

The stimulation leads to expression of an activation marker in T cells specific for the respective antigen. Consequently, in step b), antigen-specific T cell cells can be selected based on expression of the activation marker. The activation marker may be secretion of a cytokine, such as IPNγ, TNFα, or IL-10, preferably, IFNy-secretion. Alternatively, the activation marker may be one surface molecule or a combination of surface molecules, e.g., CD154, CD137, OX40, and/or CD69, in particular, for CD4+ T cells, preferably, CD154. CD137 also is a preferred activation marker.

The expression of a marker may be assayed *via* techniques such as fluorescence microscopy, flow cytometry, ELISPOT, ELISA or multiplex analyses. Selection can be, *e.g., via* a commercially available cytokine, e.g. IPNγ, secretion assay - magnetic cell enrichment and detection kit, or by flowcytometric means.

Surface molecule expression is typically assessed by adding detection antibodies to the cells, e.g., CD154, CD137, OX40, and/or CD69. In case CD154 is used, CD154 detection antibody may be added to culture at stimulation initiation or after stimulation. In the latter case, an antibody against CD40 may be added to facilitate CD154 detection.

If the human immunophilin knockout cell of the invention is a regulatory T cell (Treg), in step b), the marker is a regulatory T cell marker such as CD25, usually a combination of regulatory T cell markers including CD25. Regulatory T cells may undergo a double selection for expression of CD25 and CD4. Said selection may be carried out simultaneously or in any order. Regulatory T cells may also be selected by the combination of CD4 and CD25 or CD137, but also the absence or low expression of CD127 or CD154. Furthermore, other common Treg identity markers can be used in combination with the ones mentioned before (such as high expression of CTLA-4, and others) in addition to those mentioned. CD8 Tregs can be isolated, e.g., through combination of positive selection for CD8 and negative selection for CD28 or CD45RB.

If the human immunophilin knockout cell of the invention is an NKT cell, in step b), the cell is isolated based on expression of an NKT cell marker, preferably, based on expression of both CD56 and CD3. Said selection may be carried out simultaneously or in any order.

If the human immunophilin knockout cell of the invention is an NK cell, in step b), the cell is isolated based on expression of an NK cell marker, preferably, based on expression of CD56 and/or NKG2D, or CD16 and lack of expression of CD3. Said selection may be carried out simultaneously or in any order.

If the human immunophilin knockout cell of the invention is a γδ T cell, in step b), the cell is isolated based on expression of a γδ T cell marker, preferably, based on expression of γ and/or δ T cell receptor.

After selection, cells can be cultured in the presence of suitable cytokines, e.g., to expand them to the desired number. For example, they may be cultured in the presence of hIL-7 and hIL-15. Alternatively or additionally, they may be cultured in the presence of irradiated feeder cells. A GMP-compliant production is preferred. During expansion, cells may be split, e.g., 1:1 upon reaching confluency. Cells may be expanded, e.g., for 0-30 days, for 1-28 days, for 3-24 days, for 7-21 days or for about 7-14 days. No expansion is required if the cells numbers needed are already present in the sample comprising the isolated cells.

At a time point during culture and/or expansion, in step c), a ribonucleoprotein complex comprising Cas (e.g., Cas9) and a guide RNA, e.g., a sgRNA targeting the gene encoding the immunophilin is introduced into the cells of said selected cell composition. For example, it can be introduced after 0-30 days, for 1-28 days, for 3-24 days, for 7-21 days, or for about 7-14 days, preferably, about 7 days.

In a preferred embodiment, the immunophilin is FKBP12. Guide RNAs, preferably, Single guide (sg)RNAs targeting FKBP12 can be prepared and selected according to methods known in the art.

For example, they may target the sequence of SEQ ID NO: 1, SEQ ID NO: 2 and/or SEQ ID NO: 3. It is known in the art how such sgRNAs can be prepared, and sgRNAs are also commercially available. Exemplary sgRNAs may comprise SEQ ID NO: 14, 15 or 16. The inventors surprisingly found that excellent results are obtained when a sgRNA targeting SEQ ID NO: 1 is employed for knockout of the FKBP12 in the cells, particularly, the T cells of the invention. Thus, preferably, a single sgRNA is used for FKBP12 knockout that targets the sequence of SEQ ID NO: 1.

A guideRNA targeting SEQ ID NO: 1 may optionally comprise the spacer sequence SEQ ID NO: 14, or a sequence having at least 80% sequence identity thereto, e.g., at least 90% or at least 95% sequence identity thereto. As the skilled person knows, the guide RNA can be two RNA system comprising of a crisprRNA that includes the target specific "spacer" sequence and a tracrRNA that allows binding to the Streptococcus pyogenes Cas9 enzyme. The guide RNA can also be chimeric single guide RNA that links the crisprRNA and the tracrRNA through different linkers. The sgRNA may represent a ribonucleic acid. It may also consist of a mix of both ribonucleoside as well as of desoxynucleosides (O'Reilly et al., 2019. Nucleic Acids Research 47(2):546-558). As the skilled person knows, the spacer sequence can be modified, e.g., by reducing its 5'-end to spacer length to 17 bases, or less (Fu et al., 2014. Nat Biotechnol 32(2):279-284), increasing the spacer length (Xu et al., 2015. Genome Res 25(8): 1147-1157) and/or replacing one, or more, nucleosides within the target sequence while retaining high cutting efficacy (Dang et al., 2015 Genome Biology Art. 280) . The guide RNA may be generated through *in vitro* transcription, or it may be synthesized chemically or enzymatically.

As the skilled person knows, the efficiency of guide RNA molecules may be enhanced by use of 2O'-methyl-3'phosphothioate modifications or others (Hendel et al. 2015. Nat Biotechnology 33(9):985-989). In the experiments described herein, a chemically synthesized sgRNA was employed. However, it is also possible to use a separate tracrRNA and crisprRNA. The sgRNA may e.g. have the sequence of SEQ ID NO: 17, or alternatively, at least 70% sequence identity thereto, optionally, at least 80%, at least 90% or at least 95% sequence identity.

The invention thus also relates to use of a sgRNA targeting any of SEQ ID NO: 1-3, preferably, SEQ ID NO: 1, for preparing a cell of the invention, in particular, for preparing a pharmaceutical T cell product of the invention.

The sgRNA preferably is a synthetic sgRNA. It may be modified to increase stability, e.g., the sgRNA may comprises at least one 2O'-methyl-3'phosphothioate modification, e.g. between the first and last three nucleotides, or 2O'-methyl-3'phosphothioate modifications between the first and the last three nucleotides. Other suitable modifications are disclosed e.g., by Hendel et al. 2015. Nat Biotechnology 33(9):985-989.

Alternatively, or additionally, the immunophilin is cyclophilin A. Guide RNAs, preferably, sgRNAs targeting cyclophilin A can be prepared and selected according to methods known in the art. Optionally, the sgRNA targets the sequence of SEQ ID NO: 4, SEQ ID NO: 5, SEQ ID NO: 6 and/or SEQ ID NO: 7.

A sgRNA targeting SEQ ID NO: 4, 5, 6 or 7 may optionally comprise the spacer sequence SEQ ID NO: 20, 21, 22, 23 or 24, respectively, or a sequence having at least 80% sequence identity thereto, e.g., at least 90% or at least 95% sequence identity thereto. As described above, the spacer sequence can however be modified. In the experiments described herein, a sgRNA also taking over the function of tracrRNA was employed. However, it is also possible to use a separate tracrRNA and sgRNA. The sgRNA may e.g. have the sequence of SEQ ID NO: 24-27, or alternatively, at least 70% sequence identity thereto, optionally, at least 80%, at least 90% or at least 95% sequence identity.

Said sgRNA may also be a synthetic sgRNA, as described herein. Alternatively, it may be in vitro transcribed RNA

The guide RNA may be a chimeric RNA, so that no additional tracrRNA is required. However, instead, a suitable tracrRNA may be employed in addition to a crRNA.

Step c) is, throughout the invention, preferably carried out by electroporation of the selected and, optionally, expanded, cells. For example, about 1-5 millions T cells can be electroporated with e.g., 1-500 µg, e.g., 40-100 µg, preferably, about 30-40 µg of cas9 protein precomplexed with the sgRNA. The sgRNA may be used in an amount of about 1-50 µg, e.g., 10-40 µg or 15-30 µg, for example, about 15 µg sgRNA may be used. Chemical transfection is also possible.

The ribonucleoprotein complex leads to DNA double strand breaks at the target sequence, and consequently, introduction of mutations by the error prone cell inherent repair machinery, and loss of expression of the respective protein. As explained above, the pattern of mutation depends on the sgRNA used.

After step c), the cells may be further cultivated and, preferably, expanded. For example, said expansion may take place under the conditions explained herein. The cells may be expanded for 0-30 days, for 1-28 days, for 3-24 days, for 7-21 days, or for about 7-14 days, preferably, about 14 days. The length of the expansion depends on the number of cells required.

During said expansion, or during part of said expansion, it is possible to d) select immunophilin knockout cells by culturing the cells in the presence of an immunosuppressant agent capable of interacting with the immunophilin. Said step is however not required, as cells not comprising the knockout mutations may also be comprised in the generated cell product. If administered to a patient, said cells will however be inhibited by the calcineurin inhibitor, so they will be less efficient in performing their desired function than knockout cells.

The cells of the invention may be frozen before pharmaceutical application, preferably, in a suitable buffer, e.g., comprising a suitable amount of DMSO, such as 10%. After thawing and before administration, it may be formulated in a pharmaceutically acceptable composition, e.g., Ringer solution or phosphate buffered saline. Of course, freezing is not required but feasible. The pharmaceutical composition may, e.g., be in liquid form such as after thawing or from fresh production. It may also be in frozen form.

Typically, the cells of the invention are formulated in physiological NaCl solution for use in application via a peripheral venous access. They can be frozen and used after thawing without need for reformulation.

The invention also provides a cell or a cell population, e.g., as described herein, wherein the cell is obtainable by carrying out the method of the invention.

The invention thus provides a cell product, preferably, a T cell product (TCP) which, advantageously, is suitable for medical applications, in particular, for treatment of a human subject or patient.

As another embodiment, the invention provides a pharmaceutical composition comprising the cell of the invention or the cell population of the invention, e.g., the TCP of the invention. The pharmaceutical composition typically further comprises at least one pharmaceutically acceptable carrier, diluent or excipient. Preferably, the pharmaceutical composition comprises an isotonic salt (NaCl) solution, e.g., Ringer solution, and/or an aqueous buffer in order to stabilize the cells. As used herein, the term pharmaceutically acceptable excipient includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

The amount of cells administered to a patient depends on several factors and may be judged by the skilled person, e.g., depending on the weight, age, and condition of the patient and the specificity of the cells. For example, 1^{∗}10⁵ cells or more, 5^{∗}10⁵ cells or more, or about 1^{∗}10⁶-5^{∗}10⁹ cells, e.g., 1^{∗}10⁷-1^{∗}10⁹ or 5^{∗}10⁷-5^{∗}10⁸ cells may be administered to a patient.

A kit comprising the pharmaceutical composition of the invention and an immunosuppressant agent capable of interacting with the immunophilin is also provided by the invention, i.e., in the case of an FKBP1^{k.o.}, Tacrolimus, or in the case of an cyclophilin A^{k.o.}, CsA. Accordingly, a patient who has been administered the pharmaceutical composition may receive immunosuppressive treatment with the respective immunosuppressive agent to which the T cells of the invention are resistant, so that these T cells are not suppressed by the immunosuppressive agent. The kit may optionally further comprise an immunosuppressant capable of inhibiting an immune response of the cells, which in case any undesired effects are caused by the T cells of the invention, may be administered to said patient to reduce or prevent said undesired effects.

Regardless of whether the cells of the invention are provided in a kit or separately, the treated subject may be immunosuppressed with an immunosuppressive agent capable of interacting with the immunophilin. Said immunosuppressant may be administered simultaneously, or in any order. Typically, the subject is already immunosuppressed when the pharmaceutical composition of the invention is administered due to the time needed for preparation of the cells of the invention, if these are derived from the subject. Alternatively, in particular, if cells from a library are used, the cells may be administered first, followed by the immunosuppressive agent after engraftment, or the immunosuppressive agent may be administered first, or both may be administered essentially simultaneously, e.g., within a day. The patient may e.g. require a treatment with an antigen specific T cell product and an immunosuppressant agent, particularly a patient diagnosed with an infection (e.g., EBV, CMV, BKV, pneumonia or myocarditis), or wherein the patient has received an allogeneic transplant.

Virus-specific or tumor-specific T-cells can selectively target pathogen-infected or tumour cells. In fact, there are promising data on adoptive anti-viral or anti-cancer T-cell therapy in immunocompromised patients (e.g. against CMV, EBV, EBV-associated lymphoma). However, the cell therapy is not effective in all patients. The main reason is the reduced efficacy and survival after transfer by the negative effects of immunosuppression in those patients which cannot be reduced because of risk of alloreactivity. The pharmaceutical product of the present invention comprises cells resistant to immunosuppression mediated by the powerful immunosuppressive calcineurin inhibitors by silencing key signalling molecules of the immunosuppressive drugs. In contrast to prior art approaches used so far, which have several limitations (low efficacy, complicated manufacturing process, targeting single molecules only), the present invention provides a vector-free targeted, highly efficient and robust gene editing approach allowing easy combinations (double/triple knock-outs for different immunosuppressive drugs - CNI + steroid). Resistance to immunosuppression can also be combined with other gene manipulations, e.g. redirecting specificities by a CAR or a tgTCR, or prevention of exhaustion by PD1^{ko}.

The provision of virus-specific immunosuppressant-resistant T cells, in particular, memory/effector T-cells makes the combinatory use of those cells specific to the challenging virus (e.g. SARS-Cov2, Influenza) with the respective immunosuppressive drug possible, which further combats undesired bystander immune reactivity. In other words, this dissects protective specific anti-pathogen T-cell response from immunopathologic unspecific pathogen-related immune response.

The pharmaceutical composition or kit of the invention, wherein the T cell is a T cell specific for a virus, another pathogen or a cancer antigen, or an NK cell, typically is for use in treatment or prevention of an infection with the virus or the other pathogen or for use in treatment of the cancer. For example, the virus infection can be infection with a EBV, CMV, BKV, influenza, MERS, SARS-CoV-1 or SARS-CoV-2. The T cell of the invention, and its pharmaceutical use for treatment of the relevant infection, e.g., virus infection, may be particularly relevant in cases wherein there are no other causative treatment options available. In one embodiment, the T cell thus is a T cell specific for an antigen from SARS-CoV2.

A T cell of the invention specific for a cancer antigen, e.g., a tumor-specific antigen, a differentiation antigen, a viral antigen, or a cancer-testis antigen, for example are MAGE-A1, CTAG1B, AFP, CEA, CA-125, ETA, tyrosinase, or NY-ESO-1 but also oncogenic viruses, such as EBV, HPV, HBV, may, e.g., be useful for adoptive T cell therapy of cancer, e.g., in a patient receiving immunosuppression to suppress unwanted immune reaction, e.g. transplantation, autoimmunity etc. It may also be required, e.g., if a cancer patient requires immunosuppression to prevent rejection of a transplantation or for controlling an autoimmunity or an overwhelming immune reaction as result of cancer immunotherapy. Also, e.g., in post-transplant lymphoproliferative disorder, a calcineurin inhibitor may be provided to the patient in combination with virus-specific, e.g. EBV-specific T cells. Immunosuppression may also be part of the cancer treatment, e.g., to suppress unwanted side effects of anti-cancer immunotherapy (for example caused by treatment with checkpoint inhibitors or CAR-T cells).

As mentioned, regulatory T cells may be able to reshape undesired immune responses. Extensive preclinical studies demonstrate the power of this approach to combat undesired immune reactions.

First studies in patients demonstrate some beneficial effects of adoptively transferred regulatory T cells in transplantation, but a complete weaning of immunosuppression has not been feasible yet. Moreover, regulatory T cells are less efficient in controlling already established pathogenic effector T cell responses compared to prevention of undesired *de novo* T-cell responses. This typically makes the use of simultaneous immunosuppression necessary, not only in transplantation but also in the other diseases described above.

Again, the function and survival of regulatory T cells in the state of the art is strongly inhibited by immunosuppressive drugs like CNIs, lowering their efficacy
T cells play a central role in the key task of the immune system - maintaining the integrity of the organism, such as in infections with pathogens, the removal of transformed cancer cells and the regeneration of tissue damage. This is a complex process that is finely regulated by a balance of effector cells (proinflammatory) and regulatory cells (so-called Treg) in the course of the immune response to avoid undesired immune reactions mediating pathogenic effects. However, a misdirected or exaggerated undesired T cell response can lead to acute and chronic immunopathologies, such as autoimmunity, hyperinflammation, or damaged tissue repair up to scarring and *functio lesea.* Undesired T cell reactions can also result from a physiological but unhelpful T cell response such as alloreactivity (rejection of organ transplants, GvH disease after HSCT) or immunoreactivity against therapeutics, such as protein-based biologics, or gene and cell therapy products.

Current therapy strategies to combat undesired immune reactions are based on broad immunosuppressive drugs, such as calcineurin inhibitors, kinase inhibitors, corticosteroids, antiproliferative drugs, or more targeted inhibitors of single effector molecules, such as anti-TNF, anti-IL-1, or anti-IL-17 mAbs. The options are typically selected based on the kind and strength of undesired reactions, the long-term outcome of the disease, and the safety and efficacy profile of a particular therapy in a patient.

The most powerful immunosuppression using calcineurin inhibitors (the most powerful inhibitors of T cell function and proliferation), steroids (having very broad anti-inflammatory profile and inducing lymphocyte death) and/or antiproliferative drugs (inhibition of clonal expansion) is used in transplantation (both solid organs and HSCT) as well as in very severe autoimmune diseases or hyperinflammatory diseases (e.g. atopic eczema).

These strategies have several limitations:
- Unspecific immunosuppression targets pathogenic T cells and their downstream products, but also the protective T cell response resulting in enhanced risk of infections and tumors.
- Despite availability of a broad portfolio of immunosuppressive drugs with good efficacy in many patients, there is a significant number of non-responders to standard-of-care.
- Most immunosuppressive drugs used target not only the effector T cells but also the useful regulatory T cells, resulting in a total suppression of all T cells, both "bad and good guys", which prevents reshaping to an immune balance with sustainable therapeutic effects. This makes immunosuppression in most immunopathologic disease to a life-long maintenance therapy with adverse effects and high direct and indirect costs.

Due to these limitations, immunosuppressive approaches are standard in transplantation medicine and for severe autoimmunopathies and hyperinflammatory diseases, but are rarely used in immunopathologies caused by "bystander" T cell activation. Such immunopathologies are associated with many severe viral infections (e.g. after infections by SARS-CoV2, influenza, hepatitis viruses etc.). If these immunopathologies occur, the useful effect of immunosuppressive drugs (inhibition of undesired bystander T cell activation) so far had to be weighed against the potential danger of inhibiting the protective anti-pathogen/cancer response.

This problem is addressed by the invention, meeting a high medical need for new therapeutic approaches to combat undesired T cell reactivity with increased efficacy, sustainability, and specificity. Consequently, the present invention provides a cell of the invention that may be a regulatory T cell, for use in balancing an unwanted (undesired) immune response in a patient.

The T cell products of the invention comprising regulatory T cells may sustainably reshape a disturbed immune balance, e.g., in the cases mentioned above.

Thus, in this embodiment, the pharmaceutical composition of the invention comprising regulatory T cells is useful for balancing an undesired immune response, e.g., an immune response to a self-antigen, or an allogeneic antigen. The antigen may also be a xenogeneic antigen. The T cell may thus be useful for treatment of an autoimmune response, if the antigen is a self-antigen, or for reducing or preventing an immune response to a transplant, if the antigen is an allogeneic antigen. They may also be useful for treatment of an allergy, if the antigen is a non-self-antigen, e.g., a harmless antigen, but also in patients receiving immunosuppression to prevent immunogenicity of gene therapy (e.g. following adeno-associated vectors, CRISPR/Cas *in vivo* gene editing, CAR cell constructs). Such therapy may be combined, at least at the beginning, with an immunosuppressant therapy including the calcineurin inhibitor. During the course of the therapy, e.g., after stable establishment of the regulatory T cells in the patient, the dose of the calcineurin inhibitor may be reduced, or it may be altogether discontinued. If symptoms of the immunopathology re-appear, the immunosuppressant treatment may be re-introduced.

A cell of the invention that is a regulatory T cell specific for a defined antigen, e.g., from a virus such as hepatitis B/C, MERS, SARS-CoV1 and SARS-CoV-2, it may advantageously be used in the context of a pathologic immune response to said virus, e.g., causing a cytokine storm or symptoms of septic shock. However, antigen specificity is not required, because, as explained above, bystander activation is a well-known feature of such immunopathologies, optionally, in the context of a septic shock.

In another embodiment, a cell of the invention that is a regulatory T cell specific for an alloantigen, e.g., a particular MHC molecule expressed by a transplant such as an organ or stem cell transplant or allogeneic iPSC derived product a subject has received or is to receive, may advantageously be used in the context of application or transplantation.

The invention also provides an immunosuppressant agent capable of interacting with an immunophilin, i.e., Tacrolimus and/or cyclosporine A, for use in suppressing an undesired immune response in a patient, wherein the patient is administered a pharmaceutical composition of the invention. Ther terms immunosuppressant and immunosuppressive are used interchangeably herein. As explained above, administration can be essentially simultaneously (e.g., within a day) or in any order.

Said undesired immune response may be autoimmunity, autoinflammation, allergy, an immune response to a transplant, graft versus host disease or an immunopathology caused by bystander activation, optionally, in the context of a septic shock. The undesired immune response may, e.g., be an immunopathology caused by bystander activation, which may be caused by an infection, optionally, an infection that causes pneumonia or myocarditis.

The immunopathology caused by bystander activation may be due to a viral infection with a virus e.g., CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-2, preferably, SARS-CoV-2. With the pharmaceutical composition of the present invention comprising regulatory T cells, it is now possible to treat the patient in a causal and sustainable way, whereas, previously, said patient could not have been treated with the immunosuppressant to avoid lack of the desired immune response to the pathogen, e.g., the virus, or to a cancer.

The present invention also provides a method of treating a patient in need thereof with an effective amount of the pharmaceutical composition or kit of any of the invention, comprising a human immunophilin knockout cell that is a T cell or NK cell, wherein the immunophilin is FKBP12 or Cyclophilin A, e.g., as explained herein.

As used herein, the term "treating" or "treatment" of any disease or disorder refers, in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter. "preventing" a disease or disorder refers to reducing the incidence or the likelihood of the subject getting the disease.

Administration of the pharmaceutical composition of the invention may be e.g., parenteral, intravenous, intrapulmonary or intramuscular.

The invention provides the following embodiments:
1. A human immunophilin knockout cell that is a T cell or NK cell, wherein the immunophilin is FKBP12 or Cyclophilin A.
2. The cell of embodiment 1, wherein the cell is resistant to an immunosuppressant capable of interacting with the immunophilin.
3. The cell of any of embodiments 1 or 2, wherein the immunophilin is FKBP-12.
4. The cell of any of embodiments 1 or 2, wherein the immunophilin is cyclophilin A.
5. The cell of any of embodiments 1-4, wherein the cell is a T cell.
6. The cell of embodiment 5, wherein the T cell is a CD4+ T cell.
7. The cell of embodiment 5, wherein the T cell is a CD8+ T cell.
8. The cell of any of embodiments 5-7, wherein the cell is a T cell selected from the group consisting of a T cell specific for a virus, another pathogen or a cancer antigen.
9. The cell of embodiment 8, wherein the T cell is a virus-specific T cell, wherein the virus is selected from the group comprising CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-22.
10. The cell of embodiment 9, wherein the virus is SARS-CoV-2.
11. The cell of embodiment 9, wherein the virus is CMV
12. The cell of embodiment 9, wherein the virus is EBV.
13. The cell of embodiment 9, wherein the virus is BKV.
14. The cell of embodiment 9, wherein the virus is HPV.
15. The cell of embodiment 9, wherein the virus is ADV.
16. The cell of embodiment 9, wherein the virus is influenza virus.
17. The cell of embodiment 8, wherein the cell is a T cell specific for a cancer antigen selected from the group comprising a tumor-specific antigen, a differentiation antigen, a viral antigen, a cancer-testis antigen, wherein the cancer antigen optionally is MAGE-A1, CTAG1B, AFP, CEA, CA-125, ETA, tyrosinase, or NY-ESO-1.
18. The cell of any of embodiments 1-17, wherein the cell is a regulatory T cell, preferably, a CD4+CD25+ regulatory T cell.
19. The cell of embodiment 18, wherein the antigen is a self-antigen.
20. The cell of embodiment 18, wherein the antigen is an allogeneic antigen.
21. The cell of any of embodiments 1-4, wherein the cell is an NKT cell, an NK cell or a γδ T cell.
22. The cell of embodiment 21, wherein the cell is an NKT cell.
23. The cell of embodiment 21, wherein the cell is an NK cell.
24. The cell of any of embodiments 1-17, wherein the cell is a γδ T cell.
25. The cell of any of embodiments 1-24, comprising at least one further genetic modification.
26. The cell of any of embodiments 1-25, comprising a) a knockout of both immunophilin genes as defined in claim 1, so that the genes encoding FKBP12 and Cyclophilin are knocked out, whereas the glucocorticoid receptor gene is functional.
27. The cell of any of embodiments 1-25, further comprising b) a knockout of the glucocorticoid receptor gene, whereas the second immunophilin gene as defined in claim 1 is functional.
28. The cell of any of embodiments 1-25, comprising c) a knockout of both immunophilin genes as defined in claim 1, so that the genes encoding FKBP12 and Cyclophilin are knocked out, and of the glucocorticoid receptor gene.
29. The cell of any of embodiments 1-28, further comprising d) a transgenic TCR or a chimeric antigen receptor (CAR), optionally, a transgenic TCR.
30. The cell of any of embodiments 1-28, further comprising e) a PD1^{k.o}, HLA^{k.o.}, B2M^{k.o.}, CII-TA^{k.o.}, CD74^{k.o.}, Shp1^{k.o.}, Shp2^{k.o.}, and/or DMNT3A^{k.o.}.
31. A cell population comprising cells of any of embodiments 1-30, wherein the population is a polyclonal population of cells.
32. The cell population of embodiment 31, comprising cells with InDel mutations in the genes encoding the immunophilin, wherein, preferably, at least 10% of the cells of the population comprise said InDel mutations, optionally, at least 20%, at least 50%, at least 90% or 100%.
33. The cell population of any of embodiments 31 or 32, wherein the cell population is obtainable from gene editing of the immunophilin genes of a respective cell population obtained from a human subject, preferably, gene editing mediated by Cas, e.g., Cas9.
34. The cell population of any of embodiments 31 to 33, wherein at least 10% of the cells of the population are resistant to the action of an immunosuppressant agent capable of interacting with the immunophilin, optionally, at least 20%, at least 50%, at least 90% or 100%.
35. A method for preparing a human immunophilin knockout T cell or NK cell of any of embodiments 1-30 or a T cell or NK cell population of any of embodiments 31-34, comprising
   a) stimulating T cells or NK cells obtained from a subject,
   b) isolating stimulated T cells or NK cells based on expression of a marker to obtain a composition of selected T cells or NK cells, and
   c) gene editing the cells of said composition to knock out the gene encoding the immunophilin, preferably, introducing a ribonucleoprotein complex comprising a CRISPR associated protein (Cas) and a guide RNA, preferably, a sgRNA targeting the gene encoding the immunophilin into the T cells of said composition.
36. The method of embodiment 35, further comprising d) selecting immunophilin knockout cells by culturing the cells in the presence of an immunosuppressant agent capable of interacting with the immunophilin.
37. The method of any of embodiments 35-36, wherein the cells are T cells.
38. The method of any of embodiments 35-37, wherein the human immunophilin knockout cell is a T cell specific for a virus, another pathogen or a cancer antigen, wherein, in step a), the T cells are stimulated with a virus-derived antigen, another pathogen-derived antigen or a cancer antigen, and in step b), the marker is an activation marker selected from the group comprising IFNγ-secretion.
39. The method of any of embodiments 35-38, wherein the human immunophilin knockout cell is a regulatory T cell, wherein, in step b), the marker is a regulatory T cell marker selected from the group comprising CD25.
40. The method of any of embodiments 35-38, wherein the human immunophilin knockout cell is an NKT cell, wherein, in step b), the cell is isolated based on expression of an NKT cell marker, preferably, based on expression of CD56 and CD3.
41. The method of any of embodiments 35-38, wherein the human immunophilin knockout cell is an NK cell, wherein, in step b), the cell is isolated based on expression of an NK cell marker, preferably, based on expression of CD56 and lack of expression of CD3.
42. The method of any of embodiments 35-38, wherein the human immunophilin knockout cell is a γδ T cell, wherein, in step b), the cell is isolated based on expression of a γδ T cell marker, preferably, based on expression of γ and/or δ T cell receptor.
43. The method of any of embodiments 35-42, wherein the immunophilin is FKBP12 and the guide RNA targets the sequence of SEQ ID NO: 1, SEQ ID NO: 2 and/or SEQ ID NO: 3,
44. The method of embodiment 43, wherein a single sgRNA is used for FKBP12 knockout, targeting the sequence of SEQ ID NO: 1, wherein said sgRNA preferably comprises SEQ ID NO: 14 or a sequence having at least 80% sequence identity thereto, wherein said sgRNA optionally comprises SEQ ID NO: 17 or a sequence having at least 80% sequence identity thereto.
45. The method of any of embodiments 35-44, wherein the immunophilin is cyclophilin A and the guide RNA targets a sequence selected from the group comprising SEQ ID NO: 4-7, wherein said guide RNA optionally targets SEQ ID NO: 5 or 6.
46. The method of any of embodiments 35-45, wherein the guide RNA is a synthetic sgRNA.
47. The method of any of embodiments 35-46, wherein the guide RNA comprises at least one 2O'-methyl-3'phosphothioate modification, e.g. between the first and last three nucleotides.
48. The cell of any of embodiments 1-30 or the cell population of any of embodiments 31-34, wherein the cell is obtainable by carrying out the method of any of embodiments 35-47.
49. A pharmaceutical composition comprising the cell of any of embodiments 1-30 or the cell population of claim any of embodiments 31-34.
50. A kit comprising the pharmaceutical composition of embodiment 49 and an immunosuppressant agent capable of interacting with the immunophilin, wherein the kit optionally further comprises an immunosuppressant agent capable of inhibiting an immune response of the cells.
51. The pharmaceutical composition of embodiment 49 or the pharmaceutical kit of embodiment 50, wherein the T cell is a T cell specific for a virus, another pathogen or a cancer antigen, or an NK cell, for use in treatment or prevention of an infection with the virus or the other pathogen or for use in treatment of the cancer.
52. The pharmaceutical composition or the pharmaceutical kit for use of embodiment 51, wherein the treated subject is immunosuppressed with an immunosuppressant agent capable of interacting with the immunophilin.
53. The pharmaceutical composition of embodiment 49 or the pharmaceutical kit of embodiment 50, wherein the cell is a regulatory T cell, for use in balancing an unwanted immune response in a patient.
54. The pharmaceutical composition or the pharmaceutical kit for use of embodiment 51, wherein the patient has a condition selected from the group comprising autoimmunity, autoinflammation, allergy, a transplantation, graft versus host disease and an immunopathology due to bystander activation, optionally, in the context of a septic shock.
55. An immunosuppressant agent capable of interacting with an immunophilin selected from the group consisting of Tacrolimus and cyclosporine A for use in suppressing an undesired immune response in a patient, wherein the patient is administered a pharmaceutical composition of embodiment 49 or a pharmaceutical composition for use of any of embodiments 51-54.
56. The immunosuppressant agent for use of embodiment 55, wherein the undesired immune response is autoimmunity, autoinflammation, allergy, an immune response to a transplant, graft versus host disease or an immunopathology caused by bystander activation, optionally, in the context of a septic shock.
57. The immunosuppressant agent for use of any of embodiments 55-56, wherein the undesired immune response is an immunopathology caused by an infection that optionally causes pneumonia or myocarditis.
58. The immunosuppressant agent for use of any of embodiments 55-57, wherein the undesired immune response is an immunopathology caused by bystander activation due to a viral infection with a virus selected from the group comprising EBV, CMV, BKV, influenza, MERS, SARS-CoV-1 and SARS-CoV-2, preferably, SARS-CoV-2.
59. The immunosuppressant agent for use of any of embodiments 55-58, wherein said patient could not otherwise have been treated with the immunosuppressant to avoid lack of a desired immune response selected from an immune response to a virus, another pathogen or a cancer.
60. A method of treating a patient in need thereof with an effective amount of the pharmaceutical composition or kit of any of embodiments 49-54 or the immunosuppressant agent of any of embodiments 55-59.

The invention is further illustrated by the following examples and figures. These examples are meant to illustrate the invention but not to limit its scope.

All references cited herein are herewith fully incorporated by reference.

### Brief Description of the Figures

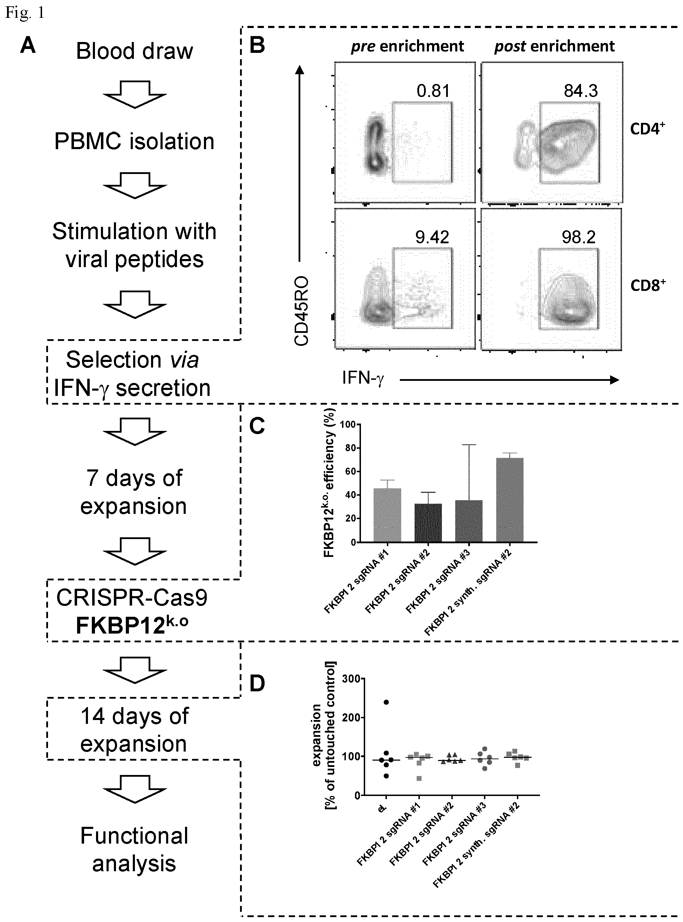

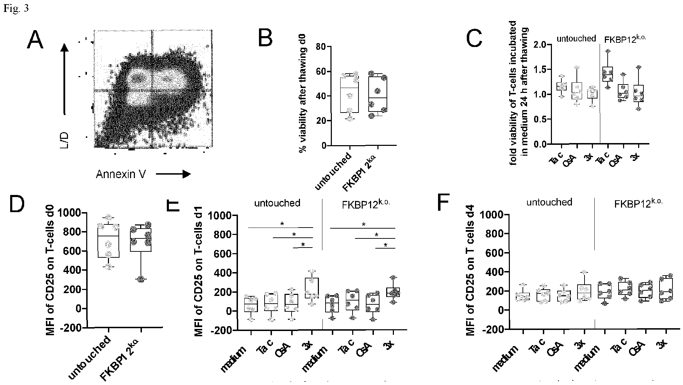

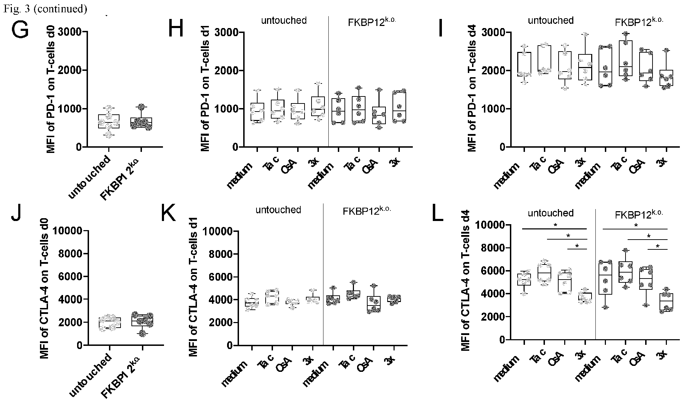

**Fig. 1****: Protocol outline for a GMP-compatible approach to induce Tacrolimus-resistance in a CMV-specific T-cell product using vector-free CRISPR-Cas9 technology**
   **a)** Individual steps of the production process.
   **b)** Representative purities of CMV-specific IFNγ-producing T-cells *pre-* and *post*-enrichment from peripheral blood mononuclear cells using an IFNy capture assay after stimulation with overlapping peptide pools of CMV_{IE-1} and CMVₚₚ₆₅ for 6 h.
   c) K.O. efficiency of FKBP12 in CMV-specific T-cell products calculated using Synthego's ICE algorithm based on Sanger-sequencing of amplified DNA of the FKBP12 gene area.
   **d)** Expansion rates of electroporated CMV-specific T-cell products normalized to expansion rates of the unmodified control from d7 to d21 (following electroporation) calculated from living cell counts determined by trypan blue staining and counting in a Neubauer counting chamber.
   **e)** Different immunosuppressants used for functional analyses and their adaptor proteins/receptors and pathways affected. Classical triple immunosuppression consists of a CNI, Prednisolone and Mycophenolic acid. Tacrolimus is used in the experiments, because it is the standard at the Charité clinic and other clinics, and it is of interest to investigate how the Tacrolimus-resistant TCP behave once injected into a triple immunosuppressed SOT recipient treated with this combination.
**Fig. 2****: Functional profiles of CMV-specific T-cell products with disrupted FKBP12** CMV-specific stimulation of T-cell products at day 21 of culture using antigen-presenting cells (autologous lymphoblastic B-cell lines) pulsed with overlapping peptide pools of CMV_{IE-1} and CMVₚₚ₆₅ (6 h stimulation). Cytokines were captured in T-cells by addition of Brefeldin A after 1 h of stimulation and cells were stained using a fixation/permeabilization kit and fluorescently labelled antibodies. Data were acquired by multicolor flow cytometry. Immunosuppressants were added where indicated at the following clinically relevant doses: Tac = 6 ng/ml Tacrolimus; CsA = 120 ng/ml Cyclosporine A; Tac/Pred/MPA = 6 ng/ml Tacrolimus + 0.57 µg/ml Prednisolone + 2.7 µg/ml Mycophenolic acid. Stars symbolize p-values < 0.05, which were determined by tests for normal distribution (Shapiro-Wilk and Kolmogorov-Smirnov tests), followed by one-way ANOVA (normally distributed data sets) or Friedman test (not normally distributed data sets) and paired t-tests (normally distributed data sets) or Wilcoxon matched pairs signed rank tests (not normally distributed data sets) as posttests.
   **a)** Representative flow cytometry plots show CD4⁺ and CD8⁺ IFNy and TNFα-producers in unmodified and FKBP12^{k.o.}-CMV-TCPs edited with synthetic sgRNA#2. Unstimulated and CMV-peptide-stimulated T-cell products are shown in the presence of the indicated immunosuppressants.
   b) Summary of CD4⁺ IFNy-producers in unmodified and FKBP12-specific sgRNA#1-3/synthetic modified sgRNA#2 gene-edited FKBP12^{k.o.}-CMV-TCPs in the presence of the indicated immunosuppressants.
   c) Summary of CD8⁺ IFNy-producers in unmodified and FKBP12-specific sgRNA#1-3/synthetic modified sgRNA#2 gene-edited FKBP12^{k.o.}-CMV-TCPs in the presence of the indicated immunosuppressants.
   **d)** Summary of CD4⁺ TNFα and IFNγ-double-producers in unmodified and FKBP12-specific sgRNA#1-3/synthetic modified sgRNA#2 gene-edited FKBP12^{k.o.}-CMV-TCPs in the presence of the indicated immunosuppressants.
   **e)** Summary of CD4⁺ TNFα/IFNγ--double-producers in unmodified and FKBP12-specific sgRNA#1-3/synthetic modified sgRNA#2 gene-edited FKBP12^{k.o.}-CMV-TCPs in the presence of the indicated immunosuppressants.
   **f)** "VITAL" assays were performed to determine killing capacity of CMV-TCPs: percentage killing of CMV-specific peptide loaded autologous LCLs normalized to a population of allogenic LCLs by CMV-specific T-cells from FKBP12^{k.o.}- and unmodified CMV-TCPs added at a 10:1 ratio in absence or presence of the indicated immunosuppressants. Killing was calculated as described previously.³⁵
**Fig. 3****: FKBP12**^{k.o.}-**CMV-T-cell products after thawing: viability, activation/exhaustion and memory markers after culture in immunosuppressants**
   Unmodified and FKBP12^{k.o.}-CMV-TCPs (edited with synthetic sgRNA#2) were frozen in FCS with 10 % DMSO (indicated by the ice symbol) and stored in liquid nitrogen until thawing in a water bath at 37°C (indicated by the orange wavy lines). Culture was performed in complete medium without cytokines with addition of the indicated immunosuppressant (Tac = 6 ng/ml Tacrolimus; CsA = 120 ng/ml Cyclosporine A; 3x = 6 ng/ml Tacrolimus + 0.57 µg/ml Prednisolone + 2.7 µg/ml MPA) at d 0 and d 2. Addition of drugs is indicated by the pipette and a green teardrop. Stars symbolize p-values < 0.05, which were determined by tests for normal distribution (Shapiro-Wilk and Kolmogorov-Smirnov tests), followed by one-way ANOVA (normally distributed data sets) or Friedman test (not normally distributed data sets) and paired t-tests (normally distributed data sets) or Wilcoxon matched pairs signed rank tests (not normally distributed data sets) as posttests.
   **a)** Representative flow cytometry plot showing L/D and Annexin V staining of single cells after thawing and demonstrating the gating strategy: double negative staining (lower left quadrant) indicates the population of viable cells.
   b) Viability of single cells in unmodified (green) and FKBP12^{k.o.}-CMV-TCPs (red) directly after thawing (d0).
   c) Viability of single cells in unmodified (green) and FKBP12^{k.o.}-CMV-TCPs (red) cultured in the indicated immunosuppressant 24 h after thawing (d1).
   **d)** MFI of CD25 on single T-cells of unmodified (green) and FKBP12^{k.o.}-CMV-TCPs (red) directly after thawing (d0).
   **e)** MFI of CD25 on single T-cells of unmodified (green) and FKBP12^{k.o.}-CMV-TCPs (red) cultured in the indicated immunosuppressant 24 h after thawing (d1).
   **f)** MFI of CD25 on single T-cells of unmodified (green) and FKBP12^{k.o.}-CMV-TCPs (red) cultured in the indicated immunosuppressant 96 h after thawing (d4).
   **g)** MFI of PD-1 on single T-cells of unmodified (green) and FKBP12^{k.o.}-CMV-TCPs (red) directly after thawing (d0).
   **h)** MFI of PD-1 on single T-cells of unmodified (green) and FKBP12^{k.o.}-CMV-TCPs (red) cultured in the indicated immunosuppressant 24 h after thawing (d1).
   **i)** MFI of PD-1 on single T-cells of unmodified (green) and FKBP12^{k.o.}-CMV-TCPs (red) cultured in the indicated immunosuppressant 96 h after thawing (d4).
   **j)** MFI of CTLA-4 on single T-cells of unmodified (green) and FKBP12^{k.o.}-CMV-TCPs (red) directly after thawing (d0).
   **k)** MFI of CTLA-4 on single T-cells of unmodified (green) and FKBP12^{k.o.}-CMV-TCPs (red) cultured in the indicated immunosuppressant 24 h after thawing (d1).
   **l)** MFI of CTLA-4 on single T-cells of unmodified (green) and FKBP12^{k.o.}-CMV-TCPs (red) cultured in the indicated immunosuppressant 96 h after thawing (d4).

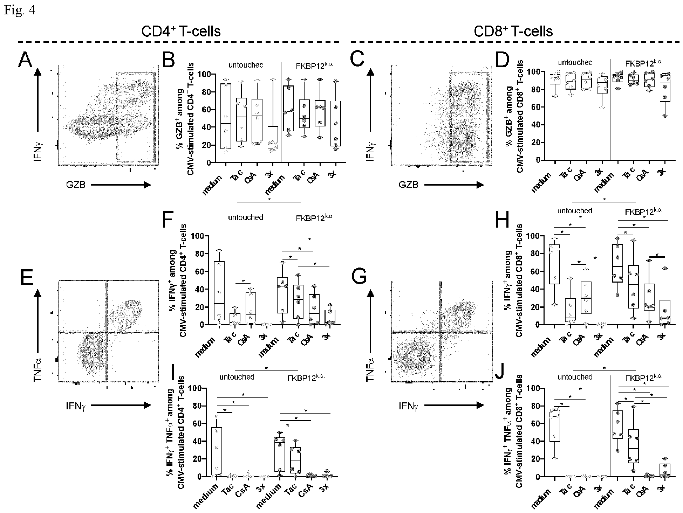

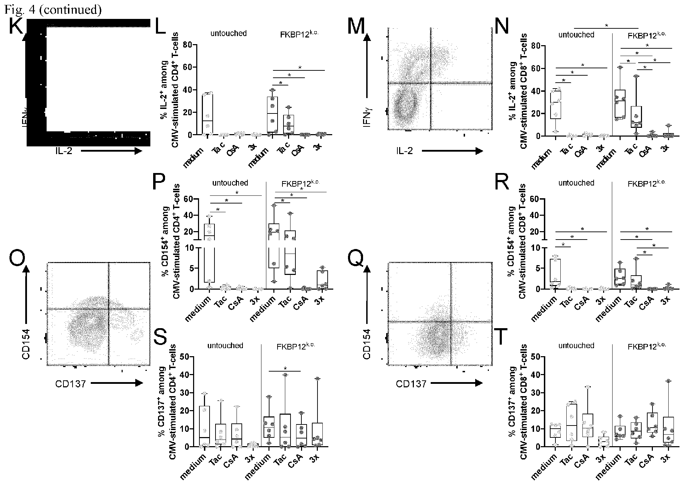

**Fig. 4****: FKBP12^{k.o.}-CMV-T-cell products after thawing: superior response to CMV-specific stimulation in presence of Tacrolimus compared to unmodified CMV-T-cell products** Unmodified and FKBP12^{k.o.}-CMV-TCPs (edited with synthetic sgRNA#2) were cultured in the indicated immunosuppressants (Tac = 6 ng/ml Tacrolimus; CsA = 120 ng/ml Cyclosporine A; 3x = 6 ng/ml Tacrolimus + 0.57 µg/ml Prednisolone + 2.7 µg/ml MPA) for 5 days after thawing. TCPs were re-stimulated with CMV-specific peptide pools delivered by autologous LCLs as antigen presenting cells for 6 h on day 5 after thawing. Autologous LCLs without peptides were added to unstimulated controls. Cytokines were captured intracellularly by addition of Brefeldin A after 1 h of stimulation. Stars symbolize p-values < 0.05, which were determined by tests for normal distribution (Shapiro-Wilk and Kolmogorov-Smirnov tests), followed by one-way Anova (normally distributed data sets) or Friedman test (not normally distributed data sets), respectively, and paired t-tests (normally distributed data sets) or Wilcoxon matched pairs signed rank tests (not normally distributed data sets), respectively, as posttests.
   **a)** Representative flow cytometry plot illustrating intracellular GZB and IFNy - staining in unstimulated (blue) and CMV-peptide stimulated CD4⁺ T-cells (red) on day 5 after thawing. The plot illustrates gating for GZB⁺ CD4⁺ T-cells.
   **b)** Summary of proportions of GZB⁺ T-cells within the CD4⁺ T-cell population (gated as illustrated in ***a*)** of unmodified (green) and FKBP12^{k.o.}-CMV-TCPs (red) following CMV-specific re-stimulation after 5 days of culture in the indicated immunosuppressants.
   c) Representative flow cytometry plot illustrating intracellular GZB and IFNy staining in unstimulated (blue) and CMV-peptide stimulated CD8⁺ T-cells (red) on day 5 after thawing. The gate illustrates gating for GZB⁺ CD8⁺ T-cells.
   **d)** Summary of proportions of GZB⁺ T-cells within the CD8⁺ T-cell population (gated as illustrated in ***c***) of unmodified (green) and FKBP12^{k.o.}-CMV-TCPs (red) following CMV-specific re-stimulation after 5 days of culture in the indicated immunosuppressants.
   **e)** Representative flow cytometry plot illustrating intracellular TNFα and IFNγ staining in unstimulated (blue) and CMV-peptide stimulated CD4⁺ T-cells (red) on day 5 after thawing. The gates illustrate gating for IFNγ⁺ (sum of the two right quadrants) and TNFα ⁺ IFNγ⁺ CD4⁺ T-cells (upper right quadrant).
   **f)** Summary of proportions of IFNγ⁺ T-cells within the CD4⁺ T-cell population (gated as illustrated in ***e***) of unmodified (green) and FKBP12^{k.o.}-CMV-TCPs (red) following CMV-specific stimulation after 5 days of culture in the indicated immunosuppressants. The background (see ***e***: blue population) was subtracted to determine exclusively CMV-specific IFNγ-producers.
   **g)** Representative flow cytometry plot illustrating intracellular TNFα and IFNγ staining in unstimulated (blue) and CMV-peptide stimulated CD8⁺ T-cells (red) on day 5 after thawing. The gates illustrate gating for IFNγ⁺ (sum of the two right quadrants) and TNFα⁺IFNγ⁺ CD8⁺ T-cells (upper right quadrant).
   **h)** Summary of proportions of IFNγ⁺ T-cells within the CD8⁺ T-cell population (gated as illustrated in g) of unmodified (green) and FKBP12^{k.o.}-CMV-TCPs (red) following CMV-specific stimulation after 5 days of culture in the indicated immunosuppressants. The background (see ***g***: blue population) was subtracted to determine exclusively CMV-specific IFNy-producers.
   **i)** Summary of proportions of TNFα⁺IFNγ⁺ T-cells within the CD4⁺ T-cell population (gated as illustrated in ***e***) of unmodified (green) and FKBP12^{k.o.}-CMV-TCPs (red) following CMV-specific stimulation after 5 days of culture in the indicated immunosuppressants. The background (see ***e***: blue population) was subtracted to determine exclusively CMV-specific TNFαIFNγ double-producers.
   **j)** Summary of proportions of TNFα⁺IFNγ⁺ T-cells within the CD8⁺ T-cell population (gated as illustrated in g) of unmodified (green) and FKBP12^{k.o.}-CMV-TCPs (red) following CMV-specific stimulation after 5 days of culture in the indicated immunosuppressants. The background (see g: blue population) was subtracted to determine exclusively CMV-specific TNFα⁺IFNγ⁺ double-producers.
   **k)** Representative flow cytometry plot illustrating intracellular IL-2 and IFNy staining in unstimulated (blue) and CMV-peptide stimulated CD4⁺ T-cells (red) on day 5 after thawing. The gates illustrate gating for IL-2⁺ (sum of the two right quadrants).
   **l)** Summary of proportions of IL-2⁺ T-cells within the CD4⁺ T-cell population (gated as illustrated in ***k***) of unmodified (green) and FKBP12^{k.o.}-CMV-TCPs (red) following CMV-specific stimulation after 5 days of culture in the indicated immunosuppressants. The background (see ***k***: blue population) was subtracted to determine exclusively CMV-specific IFNy-producers.
   **m)** Representative flow cytometry plot illustrating intracellular IL-2 and IFNγ staining in unstimulated (blue) and CMV-peptide stimulated CD8⁺ T-cells (red) on day 5 after thawing. The gates illustrate gating for IL-2⁺ (sum of the two right quadrants).
   **n)** Summary of proportions of IL-2⁺ T-cells within the CD8⁺ T-cell population (gated as illustrated in ***m***) of unmodified (green) and FKBP12^{k.o.}-CMV-TCPs (red) following CMV-specific stimulation after 5 days of culture in the indicated immunosuppressants. The background (see ***m***: blue population) was subtracted to determine exclusively CMV-specific IFNγ-producers.
   **o)** Representative flow cytometry plot illustrating intracellular CD154 and CD137 staining in unstimulated (blue) and CMV-peptide stimulated CD4⁺ T-cells (red) on day 5 after thawing. The gates illustrate gating for CD137⁺ (sum of the two right quadrants) and CD154⁺ CD4⁺ T-cells (sum of the two upper quadrants).
   **p)** Summary of proportions of CD154⁺ T-cells within the CD4⁺ T-cell population (gated as illustrated in ***o***) of unmodified (green) and FKBP12^{k.o.}-CMV-TCPs (red) following CMV-specific stimulation after 5 days of culture in the indicated immunosuppressants. The background (see ***o***: blue population) was subtracted to determine exclusively CMV-specific CD154⁺ CD4⁺T-cells.
   **q)** Representative flow cytometry plot illustrating intracellular CD154 and CD137 staining in unstimulated (blue) and CMV-peptide stimulated CD8⁺ T-cells (red) on day 5 after thawing. The gates illustrate gating for CD137⁺(sum of the two right quadrants) and CD154⁺ CD8⁺ T-cells (sum of the two upper quadrants).
   **r)** Summary of proportions of CD154⁺ T-cells within the CD8⁺ T-cell population (gated as illustrated in ***q***) of unmodified (green) and FKBP12^{k.o.}-CMV-TCPs (red) following CMV-specific stimulation after 5 days of culture in the indicated immunosuppressants. The background (see ***q***: blue population) was subtracted to determine exclusively CMV-specific CD154⁺ CD8⁺T-cells.
   **s)** Summary of proportions of CD137⁺ T-cells within the CD4⁺ T-cell population (gated as illustrated in o) of unmodified (green) and FKBP12^{k.o.}-CMV-TCPs (red) following CMV-specific stimulation after 5 days of culture in the indicated immunosuppressants. The background (see ***o**:* blue population) was subtracted to determine exclusively CMV-specific CD137⁺ CD4⁺T-cells.
   **t)** Summary of proportions of CD137⁺ T-cells within the CD8⁺ T-cell population (gated as illustrated in ***q***) of unmodified (green) and FKBP12^{k.o.}-CMV-TCPs (red) following CMV-specific stimulation after 5 days of culture in the indicated immunosuppressants. The background (see ***q***: blue population) was subtracted to determine exclusively CMV-specific CD137⁺ CD4⁺T-cells.

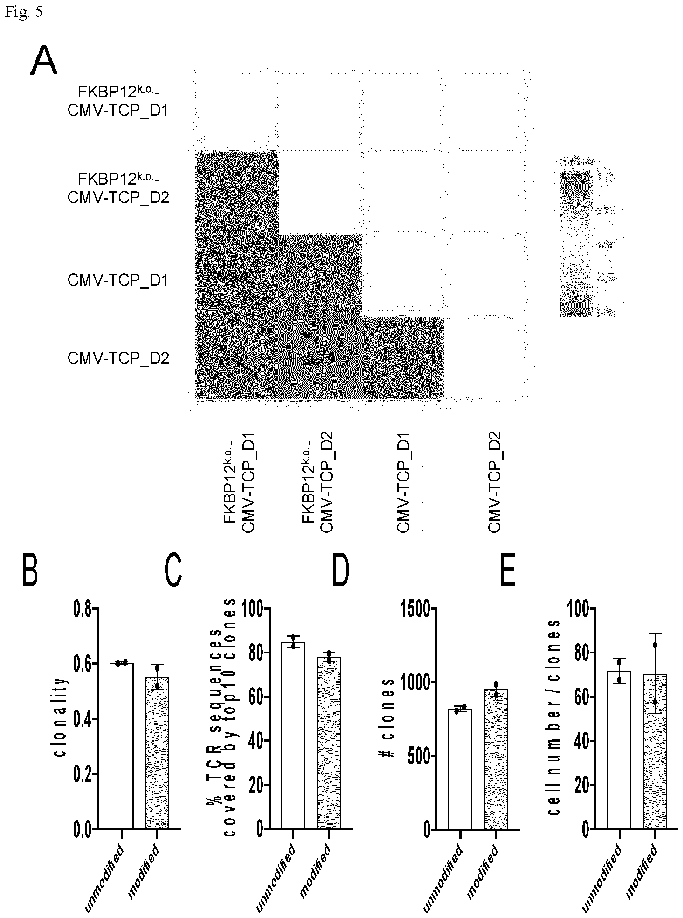

**Fig. 5****: FKBP12**^{k.o.}**-CMV-T-cell products and unedited products have a similar TCRβ repertoire**
   **a)** Similarity matrix based on the Morisita index for FKBP12^{k.o.}-CMV-TCPs and CMV-TCPs. n=2 (D1 and D2).
   **b)** Clonality of FKBP12^{k.o.}-CMV-TCPs and CMV-TCPs. n=2. A clonality of 1 would signify a product consisting of only one clone (monoclonal), the lower the clonality, the more diverse the clonal composition of the product. The clonality is the inverse of the normalized Shannon's entropy.
   **c)** Proportion of total productive frequencies covered by the top 10 most represented clones. n=2.
   **d)** Total number of clones. n=2.
   **e)** Total productive frequencies divided by the number of clones.
**Fig. 6****: Cas9 protein is undetectable in FKBP12^{k.o.}-CMV-T-cell products**
   Proportions of T-cells in TCPs that stained positive for Cas9-specific fluorescently labelled antibodies at different time points after electroporation.
**Fig. 7****:** Illuminated agarose gel after electrophoresis shows PPIA-exon 1 PCR amplicons of untouched T cells as well as T cells electroporated with a control sgRNA-Cas9 complex (CD45 sgRNA 1, Gundry et al 2016) or four sgRNA-Cas9 mix targeting PPIA exon 1 (SEQ ID NO: 4-7). PPIA-specific CRISPR-Cas9 complexes efficiently introduced deletions in exon in three biological replicates (different healthy donors). Lanes 1: D1: untouched; 2: D1: control sgRNA; 3: D1: 4xPPIA sgRNA; 4: D2: control sgRNA; 5 D2 4xPPIA sgRNA; 6: D3: control sgRNA; 7: D3: 4xPPIA sgRNA

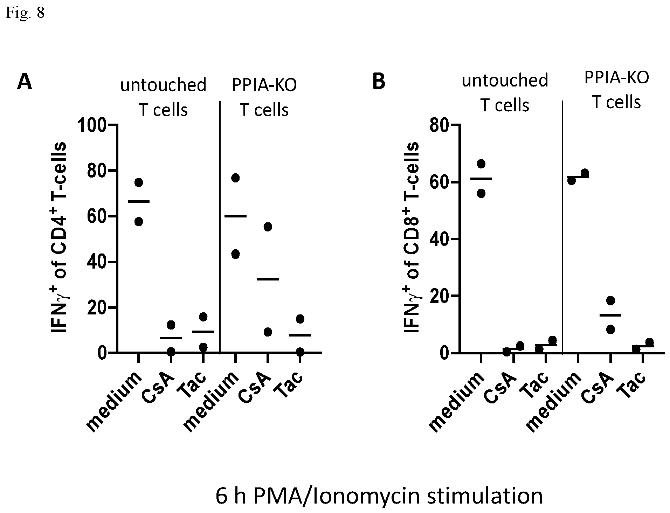

**Fig. 8****:** Therapeutic doses of calcineurin inhibitors efficiently prevent cytokine production in untouched wildtype T cells. In contrast, T cells with PPIA-KO partially retained ability to produce IFN-gamma in in the presence of Cyclosporine A, but remained unable to produce IFNy in the presence of the calcineurin inhibitor Tacrolimus. Rescue of IFNγ production was more pronounced in CD4 T cells with PPIA-KO (A) compared to the CD8 T cells with PPIA-KO (B).
**Fig. 9****: Functionality of gene-edited FKBP12-/- Treg products**
   Cells were prepared as described in Example 3. After expansion for 21 days, the cells were analysed and compared with untouched Tregs regarding expansion rate and final yield (A+B), gene editing efficacy measured by Sanger sequencing and ICE analysis (Synthego) (C) and epigenetic identity, through quantification of demethylation within the Treg-specific demethylation region (TSDR) (D). FKBP12-KO could be performed with high efficiency and even increased overall yield, presumably due to reduced inhibition by rapamycin treatment. FKBP12-KO Treg retained epigenetic identity of natural Treg as indicated by high TSDR demethylation. The cells are thus stable.

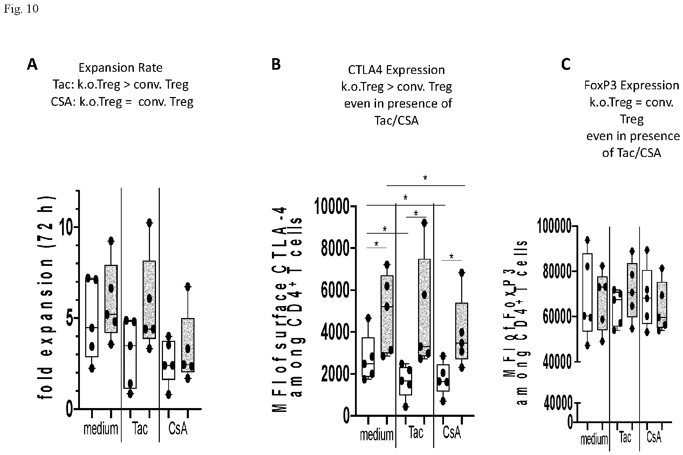

**Fig. 10****: Functional k.o.: FKBP12-/Treg are resistant to Tac but still sensitive to CSA (for rescue)** Exemplary regulatory T cells, expanded and treated as described in Figure Z. Edited and control Treg cells were subsequently expanded in medium with or without different calcineurin inhibitors and subsequently analysed for cell expansion (A) and with flow cytometry (B+C). As expected, Tacrolimus treatment reduced the proliferation of untouched Tregs, while FKBP12-KO Tregs retained fully proliferative capacity despite the drug treatment (A). FKBP12-KO Treg displayed higher expression of the inhibitory receptor CTLA4 (B). CTLA4 is known to suppress T cells and modulate pro-inflammatory responses by dendritic cells. Further, Foxp3 transcription factor expression assessed by flow cytometry was comparable between wildtype and FKBP12-KO, with a trend toward increased Foxp3 expression of FKBP12KO-Treg in the presence of Tacrolimus indicating potential for increased stability (C). Light bars are untouched expanded Treg, dark bars are FKBP12^{k.o.} expanded Treg. Tac was used at 6 ng/ml, CsA was used at 125 ng/ml, which corresponds to pharmacological doses that may, e.g., found in patient serum.

### Tables

**Table 1: Target sequences and sequences of exemplary FKBP12-specific guide RNAs (sgRNA targeting SEQ ID NO: 1 is preferred).**

| | DNA Target sequence of sgRNA (SpCas9) | Spacer sequence of sgRNA | Full sequence of sgRNA (with standard scaffold sequence for SpCas9) |
|---|---|---|---|
| sgRN A#1 | GCACTACAC- CGGTGAG- TCGG (SEQ ID NO: 2) | GGACUACAC- CGGUG- AGUCGG (SEQ ID NO: 15) | GCACUACACCGGUGAGUCGGUUUUA- GAGCUAGAAAUAGCAAGUUAAAAU- AAGGCUAGUCCGUUAUCAACUUGAAAAA GUGGCACCGAGUCGGUGC (SEQ ID NO: 18) |
| sgRN A#2 | GGGCGCAC- CTTCCCCAA- GCG (SEQ ID NO: 1) | GGGCGCAC- CUUCCCCAA- GCG (SEQ ID NO: 14) | GGGCGCACCUUCCCCAAGCGGUUUUA- GAGCUAGAAAUAGCAAGUUAAAAU- AAGGCUAGUCCGUUAUCAACUUGAAAAA GUGGCACCGAGUCGGUGC (SEQ ID NO: 17) |
| sgRN A#3 | CGGTGAG- TAG- TGGCGCGCGG (SEQ ID NO: 3) | GGGUG- AGUAGUG- GCGCGCGG (SEQ ID NO: 16) | CGGUGAGUAGUGGCGCGCGGUUUUA- GAGCUAGAAAUAGCAAGUUAAAAU- AAGGCUAGUCCGUUAUCAACUUGAAAAA GUGGCACCGAGUCGGUGC (SEQ ID NO: 19) |

**Table 2: Target sequences and sequences of exemplary cyclophilin A-specific guide RNAs (sgRNA targeting SEQ ID NO: 5 and/or 6 is preferred).**

| | DNA Target sequence of sgRNA (SpCas9) | Spacer sequence of sgRNA | Full sequence of sgRNA (with standard scaffold sequence for SpCas9) |
|---|---|---|---|
| sgRN A#4 | CGTGG- TATAAAAGGG GCGGG (SEQ ID NO: 4) | GGUG- GUAUAAAAGG GGCGGG (SEQ ID NO: 20) | CGUGGUAUAAAAGGGGCGGGUUUUA- GAGCUAGAAAUAGCAAGUUAAAAU- AAGGCUAGUCCGUUAUCAACUUGAAAA AGUGGCACCGAGUCGGUGC (SEQ ID NO: 24) |
| sgRN A#5 | CGGCGGTGGC GTCTGCAAAA (SEQ ID NO: 5) | GGGCGGUG- GCGUCUG- CAAAA (SEQ ID NO: 21) | CGGCGGUGGCGUCUGCAAAAGUUUUA- GAGCUAGAAAUAGCAAGUUAAAAU- AAGGCUAGUCCGUUAUCAACUUGAAAA AGUGGCACCGAGUCGGUGC (SEQ ID NO: 25) |
| sgRN A#6 | CGCCGCCCGCC CGACCTCAA (SEQ ID NO: 6) | GGCCGCCCGC CCGACCUCAA (SEQ ID NO: 22) | CGCCGCCCGCCCGAC- CUCAAGUUUUAGAGCUAGAA- AUAGCAAGUUAAAAUAAGGCUAGUC- CGUUAUCAACUUGAAAAAGUGGCACCG AGUCGGUGC (SEQ ID NO: 26) |
| sgRN A#7 | GCCGCGTCTCC TTTGAGGTC (SEQ ID NO: 7) | GCCGCGUCUC CUUUGAGGUC (SEQ ID NO: 23) | GCCGCGUCUCCUUUGAGGUCGUUUUA- GAGCUAGAAAUAGCAAGUUAAAAU- AAGGCUAGUCCGUUAUCAACUUGAAAA AGUGGCACCGAGUCGGUGC (SEQ ID NO: 27) |

### EXAMPLES

### Example 1 - FKBP12^{k.o.} T cells

### Results

### Integration of FKBP12 knockout into the clinical protocol for CMV-specific T-cell product generation

The inventors adapted a vector-free protocol for electroporation and ribonucleoprotein-based knockout³⁰ of FKBP12 to make a virus-specific Tacrolimus-resistant TCP (FKBP12^{k.o.}-CMV-TCP)¹⁹ (**Fig. 1a**)**.** For this, CMV-specific T-cells were isolated from peripheral blood mononuclear cells (PBMCs), based on their interferon (IFN)γ secretion¹³, after activation by CMV_{IE-1/pp-65} overlapping peptide pools (**Fig. 1b****)**. These cells were expanded for one week. FKBP12 knockout was achieved by electroporation with ribonucleoprotein complexes of Cas9 and different sgRNAs, selected based on specificity *in silico*-prediction (**Fig. 1c**)^{31, 32}. The sgRNAs used, targeting SEQ ID NO: 1, 2 and 3, and comprising SEQ IS Nos 17-19, differed according to the respective target sequence within the coding gene for FKBP12 and the mode of production (*in vitro* transcribed *vs*. fully synthetic [synt.]).

The knockout efficiencies differed between the sgRNAs tested. For GMP compatibility reasons, we investigated one synt. sgRNA with 2O'-methyl-3'phosphothioate modifications in the first and last 3 nucleotides, which are reported to enhance editing frequencies in T-cells³³. Indeed, the synthetic version of sgRNA#2 improved k.o. efficacy of the sgRNA (**Fig. 1c**). The sgRNA#2 (targeting SEQ ID NO: 1) was chosen to be produced as a GMP compliant fully synthetic sgRNA due to its superior function in maintaining T-cell function in the presence of Tacrolimus compared to the other two sgRNAs (**Fig. 2** etc). We adapted an existing electroporation protocol for polyclonal T-cells³⁰ to our CMV-TCPs using an electroporation device suitable for GMP applications, using closed cartridges and electroporation conditions that preserved similar expansion rates to unmodified controls (**Fig. 1d**). The expansion rates are normalized to the unmodified controls, which did not undergo electroporation, to determine whether the electroporation/gene editing procedures altered the expansion capability of the cells (**Fig. 1d**). The expansion rate of the electroporated and gene edited cells was not different to the control, indicating that the electroporation procedure and gene editing did not influence the overall cell expansion rate. (**Fig. 1d**).

For functional analyses, we exposed TCPs to clinical doses of immunosuppressive drugs (**Fig. 1e**). The calcineurin inhibitor (CNI) Tacrolimus, which requires FKBP12 as an adaptor protein (**Fig. 1e**), is the main target in this project. To confirm specificity of the Tacrolimus-targeted approach, we used an alternative CNI, CsA, which depends on cyclophiloinA/Peptidylprolyl isomerase A (PPIA) as its adaptor protein (**Fig. 1e**). To test the functionality of the modified T-cells in the clinical context of solid organ transplant (SOT) recipients receiving triple immunosuppression (IS), we used classical triple IS consisting of Tacrolimus, Prednisolone (requiring the glucocorticoid receptor, which acts itself as transcription factor) and Mycophenolic acid, which directly acts on nucleotide synthesis (**Fig. 1e**).

### Cytokine production in the presence of Tacrolimus is rescued by FKBP12^{k.0.} in T-cell products

A major function of effector T-cells is the production of anti-viral cytokines to instruct other immune cells and induce an antiviral state in infected cells. Producers of multiple cytokines show particularly high antiviral functionality³⁴. Upon CMV-specific re-stimulation on day 21 of culture, we recorded similar proportions of producers of the antiviral cytokines IFNy and TNFα among CD4⁺ and CD8⁺ T-cells in FKBP12^{k.o}-CMV- and unmodified CMV-TCPs (**Fig. 2a****-e**). Addition of immunosuppressive drugs during the stimulation significantly decreased IFNγ and TNFα /IFNγ -double-producers among CD4⁺ (**Fig. 2b****, d**) and CD8⁺ T-cells (**Fig. 2c****, e**), which was partially rescued by FKBP12^{k.o.} (**Fig. 2b****-e**). Although cytokine production improved in all FKBP12^{k.o.} conditions in the presence of a clinically relevant dose of Tacrolimus, only samples electroporated with sgRNA#2 retained physiological levels of cytokine producers in stimulated TCPs (**Fig. 2b****-e**). We therefore tested sgRNA#2 as a GMP-compliant synthetic modified sgRNA, with 2O'-methyl-3'phosphothioate modifications in the first and last 3 nucleotides, which are reported to enhance editing frequencies in T-cells³³. Using synthetically modified sgRNA#2, we achieved higher editing efficacy (**Fig. 1c**) and better recovery of cytokine production, especially in the presence of clinically relevant doses of triple IS, during stimulation (**Fig. 2a****-e**).

### FKBP12^{k.o.}-CMV-TCPs demonstrate comparable killing capacity to unmodified CMV-TCPs

Since antiviral T-cells induce targeted elimination of infected cells by cytotoxicity, we tested the cytotoxic killing capacity of TCPs^{35, 36}. Unlike antiviral cytokines, which were strongly affected by short-term classical immunosuppressive treatment (**Fig. 2a****-e**), the T-cell mediated killing of CMV-peptide loaded target cells was unaffected by short-term incubation with immunosuppressive drugs at clinical doses (**Fig. 2f**). Notably, the data demonstrated no differences in killing capacity of FKBP12^{k.o}-CMV-TCPs compared to unmodified CMV-TCPs, irrespective of the applied sgRNA.

### Enhanced survival of FKBP12^{k.o.} CMV-specific T-cell products in presence of Tacrolimus

Before clinical application, antiviral TCPs are typically cryopreserved until all release criteria and safety tests are accomplished. Therefore, we froze all edited and non-edited CMV-TCPs. We focused on synthetic sgRNA#2 in subsequent experiments, as it was the most effective (**Fig. 2**) and preferred GMP compliant material. Synthetic sgRNA#2-treated FKBP12^{k.o}-CMV- and unmodified CMV-TCPs were thawed and evaluated for viability and functionality post-thawing (**Fig. 3a**). The viability of the T-cells did not differ directly after thawing (d0; **Fig. 3b**), however, we found a slightly bigger proportion of living cells in thawed FKBP12^{k.o}-CMV-TCPs compared to unmodified CMV-TCPs following 24h incubation with Tacrolimus (**Fig. 3c**; p = 0.06, normalized to culture in medium without immunosuppressants). Of note, 24 h incubation with other immunosuppressants revealed no differences in viability (**Fig. 3c**).

### Exhaustion and phenotypic memory markers of FKBP12^{k.o.}- and unmodified CMV-specific T-cell products following 4 days of consecutive culture in the presence of immunosuppressants

We evaluated the cell surface expression of distinct phenotypic markers for T-cell activation (CD25) and exhaustion (programmed death (PD)-1 and Cytotoxic T-lymphocyte-associated Protein (CTLA)-4 (CTLA-4)) in the presence of clinical doses of immunosuppressants at 0, 1- and 4-days post-thawing by flow cytometry. We detected high CD25 expression directly after thawing (**Fig. 3d**), which diminished after 24 h (**Fig. 3e-f**). The mean fluorescence intensity (MFI) of CD25 remained significantly higher in TCPs cultured for 24 h with clinical doses of classical triple IS, irrespective of whether FKBP12 was genetically edited or not (**Fig. 3d**). However, no differences in CD25 expression were recorded following 4 days of culture in the presence of immunosuppressants (added every 48 h) (**Fig. 3f**). Next, we assessed exhaustion markers in the cell products. To this end, we determined the expression of PD-1 and CTLA-4 during 4 days of culture post-thawing. For all conditions, PD-1 and CTLA-4 expression increased equally over the observed time course (**Fig. 3g-****l**). Interestingly, CTLA-4 protein levels (MFI) were significantly lower in TCPs treated with triple IS irrespective of FKBP12 editing (**Fig. 3l**).

### Functional assessment of CMV-specific T-cell products exposed to Tacrolimus

Next, we assessed the functional capacity of TCPs for virus-specific effector functions after the freezing/thawing process and 5-day culture in immunosuppressants, similar to the environment faced upon injection into an immunosuppressed patient. To this end, TCPs were stimulated with CMV-specific peptide pools and assessed for intracellular accumulation of cytokines as well as the presence of activation markers.

Granzyme B (GZB) mediates apoptosis of virus-infected target cells in conjunction with perforin. Therefore, we measured intracellular accumulation of GZB in TCPs upon CMV-specific re-stimulation and found a stimulation-dependent increase of GZB in CD4⁺T-cells (**Fig. 4a**). Notably, the majority of CD8⁺ T-cells presented with pre-formed GZB even before re-stimulation (**Fig. 4c**). Neither gene-editing nor culture with immunosuppressants at clinical doses impacted the proportion of CMV-stimulated GZB⁺ CD4⁺ (**Fig. 4b**) or GZB⁺ CD8⁺ T-cells (**Fig. 4d**) in the CMV-TCPs. Next, we measured IFNγ and TNFα-production in response to CMV-specific stimulation after thawing of the TCPs and culture in immunosuppressants (**Fig. 4e****-j).** CD4⁺ and CD8⁺ T-cells produced IFNγ in response to CMV-stimulus after culture with Tacrolimus, although IPNγ-producers among CD4⁺ and CD8⁺ T-cells were significantly more frequent in FKBP12^{k.o.}-CMV-TCPs compared to unmodified CMV-TCPs after culture with Tacrolimus (**Fig. 4f****, h**). Remarkably, CD4⁺ and CD8⁺ IFNγ/TNFα-double-producers were also significantly more abundant among CD4⁺ and CD8⁺ T-cells in FKBP12^{k.o.}- compared to unmodified CMV-TCPs, in which they were almost absent (**Fig. 4i****, j**). CD8⁺ IFNγ/TNFα-double-producers were significantly more frequent among CD8⁺ T-cells from FKBP12^{k.o.}-CMV-TCPs cultured in Tacrolimus compared to CsA, which was not evident with unmodified CMV-TCPs (**Fig. 4j**). The tendency of enhanced cytokine producers among cells cultured in Tacrolimus *vs*. cell cultured in Cyclosporine A was also observed in CD4⁺ IFNγ/TNFα-double-producers and both CD4⁺ and CD8⁺ IFNγ producers from FKBP12^{k.o.}-CMV-, but not unmodified TCPs (**Fig. 4f, h, i**). In contrast, in unmodified TCPs, IFNγ producers were more frequent among the CsA-treated compared to the Tacrolimus-treated cultures (**Fig. 4f****, h**). CD4⁺ and CD8⁺ IPNγ-producers (**Fig. 4f****, h**) and CD8⁺ IFNγ/TNFα-double-producers (**Fig. 4j**) were more frequent in cultures of FKBP12^{k.o.}-CMV-TCPs cultured in triple IS compared to their unmodified counterpart.

IL-2 is mainly produced by memory T-cells with a low differentiation state and high proliferative potential, which contribute to the establishment of long-term memory³⁷. A fraction of CD4⁺ (**Fig. 4k**) and CD8⁺ T-cells (**Fig. 4m**) in thawed TCPs produced IL-2 upon CMV-specific stimulation. CD4⁺ IL-2-producers were more frequent in FKBP12^{k.o.}-CMV-TCPs cultured in Tacrolimus, compared to the corresponding unedited CMV-TCPs (**Fig. 4l**). This observation of increased IL-2 producers in FKBP12^{k}-CMV-TCPs cultured in Tacrolimus, compared to the corresponding unedited CMV-TCPs, was significant for CD8⁺ IL-2-producers (**Fig. 4n**). CD8⁺ IL-2-producers from FKBP12^{k}-CMV-TCPs cultured in Tacrolimus were significantly more frequent compared to CD8⁺ IL-2 producers from FKBP12^{k}-CMV-TCPs cultured in CsA or triple immunosuppression (**Fig. 4n**).

Next, we assessed the expression of the activation markers CD154 (CD40L) and CD137 (41BB) on CMV-stimulated TCPs. CD154 increases the activation of antigen presenting cells and is important for antibody production by B-cells³⁸. CD154 is also induced upon antigen recognition by the TCR³⁹, and is reported to be expressed on T-cells protective against CMV⁴⁰. We found CMV-specific CD154 upregulation on CD4⁺ (**Fig. 4o**), but also on a subset of CD8⁺ T-cells (**Fig. 4q**). CD154-expressing CD4⁺ (**Fig. 4p**) and CD8⁺ T-cells (**Fig. 4r**) were more frequent among FKBP12^{k.o.}-CMV-TCPs cultured in Tacrolimus compared to their unmodified counterpart (**Fig. 4p****, r**). After culture in triple IS, CD154-expressing CD4⁺ T-cells increased in FKBP12^{k.o.}- compared to unmodified CMV-TCPs upon CMV-specific stimulation (**Fig. 4p**). After culture in CsA and triple IS, CMV-specific CD154 expression by CD8⁺ T-cells in FKBP12^{k.o.}-CMV-TCPs was significantly reduced compared to Tacrolimus-treated cultures of FKBP12^{k.o.}-CMV-TCPs (**Fig. 4r**). CD137 (41BB) is another antigen-dependent activation marker on T-cells (**Fig. 4o****, q**). Except for a significant reduction in CMV-specific CD137-expressing CD4⁺ T-cells from FKBP12^{k.o.}-CMV-TCPs upon culture in CsA compared to culture without immunosuppressants (**Fig. 4s**), we did not detect differences in CMV-specific CD137-expression on T-cells among CMV-TCPs, neither after culture in immunosuppressantsnor upon FKBP12k.o.(**Fig. 4o, q, s, t**).

Taken together, these results show that, after culture in Tacrolimus, FKBP12^{k.o.}-CMV-TCPs mediate superior effector function and activation compared to unedited CMV-TCPs. The results thus indicate that FKBP12^{k.o.}-CMV-TCPs have acquired resistance to Tacrolimus.

### FKBP12^{k.o.}CMV-TCPs have a similar TCRβ repertoire as CMV-TCPs after expansion

We performed TCRβ-sequencing to compare the clonality and number of clones included in the expanded FKBP12^{k.o.}- *vs*. CMV-TCPs. There was no similarity between the TCRβ repertoires of different donors defined by the Morisita index (**Fig. 5a**). However, we detected high similarity between FKBP12^{k.o.}- and CMV-TCPs generated from the same donor (**Fig. 5a**). We detected comparable clonality (**Fig. 5b**), percentage of all TCRβ sequences covered by the top 10 clones (**Fig. 5c**) and total numbers of clones represented (**Fig. 5d**) in FKBP12b^{k.o.}-*vs*. CMV-TCPs. Hence, we conclude that gene editing did not drastically skew the TCRβ repertoire or induce excessive clonal expansion.

### Cas9 protein is undetectable in edited T-cell products after 5 days of culture

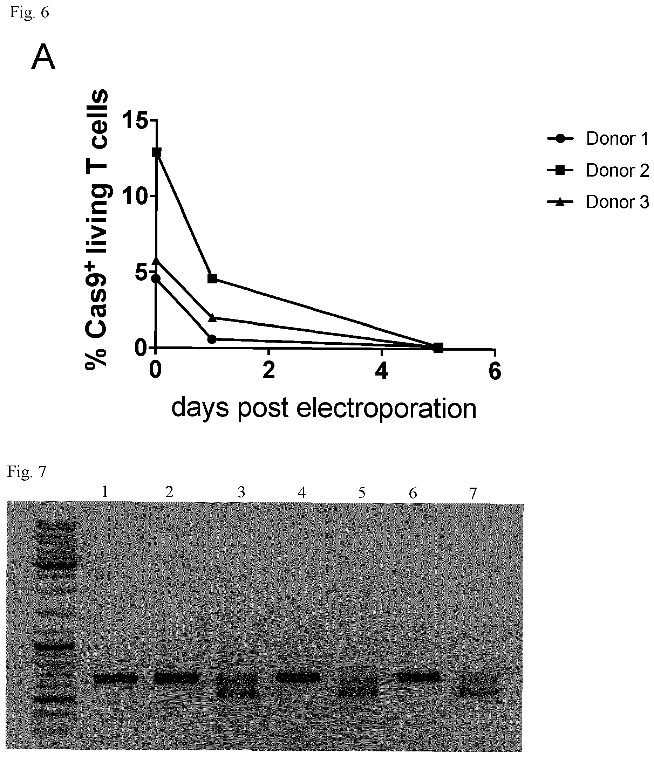

Studies on human T-cell reactivity towards the Cas9 protein^{41, 42} may raise the concern that Cas9-edited cells are potentially immunogenic. Therefore, we stained edited T-cells for Cas9 protein after electroporation. We detected Cas9 protein expression in low proportions of T-cells, which progressively decreased over time. Cas9 protein was undetectable by day 5 post-electroporation (Fig. 6). Thus, T cells of the invention may or may not comprise Cas9 protein, depending on the time point after electroporation they are used.

### Differentiation states of the different T-cell products at different stages in production

The majority of the CD8⁺ T-cells have a terminally differentiated phenotype after the isolation of CMV-reactive T-cells (CCR7⁻ CD45RA⁺), and thus, may arise the concern that these TCPs have limited longevity (**Fig. 1b**). In fact, after expansion and thawing this population was almost undetectable in the majority of TCPs, allowing the assumption that less differentiated memory T-cells expanded and represent the mass of the expanded TCP (data not shown). Knockout of FKBP12 did not have a major impact on the T-cell memory subset composition of the TCPs and there was no clear trend towards a particular subset preference (data not shown).

### Discussion

The invention provides a GMP-compliant approach for targeted gene editing of specific T-cells to achieve Tacrolimus or CsA resistance. This approach can be used, e.g., to optimize antiviral T-cell therapy for use in a setting requiring immunosuppression, for example SOT. The method of the invention comprises an antigen-specific T-cell isolation step²⁷, which is superior in terms of safety to other published protocols that start with a mixed PBMC population^{26, 28}. This safety step is important to exclude allograft-reactive IS-resistant T-cell contamination, that could cause damage to the transplanted organ. The invention provides a functional FKBP12^{k}-CMV-TCP with superior cytokine production and activation in the presence of Tacrolimus and triple IS (used in SOT) compared to unedited CMV-TCPs. The retained sensitivity of the gene-edited FKBP12k.o.-CMV-TCPs to CsA represents an important safety switch. CsA is a clinically approved drug that may be used to limit any toxicity possibly associated with the Tacrolimus-resistant FKBP12^{k.o.}-CMV-TCP in case it occurs. The method of the invention utilizes a cartridge-based GMP-compatible electroporation system and adapted electroporation conditions. Although gene knockouts using multiple sgRNAs are more efficient³⁰, immunophilin, e.g., FKBP12, knockout using a single sgRNA is preferred to minimize potential off-target effects and to ensure safety and GMP-compatibility. Moreover, advantages of a GMP-compatible synthetic sgRNA with 20'-methyl-3'phosphothioate-bonds³³ were shown. The synthetic sgRNA enhanced knockout efficiency and functionality of the product in the presence of Tacrolimus and triple IS. Recently, a similar process was reported for production of glucocorticoid-resistant antiviral T-cells^{43, 71}. All material used for the described process is either available at GMP-grade or has sufficient documentation and quality to be accepted by regulatory authorities for GMP production. This qualifies the whole process as a GMP-compliant ready to translate process. We detected no impact of classical immunosuppressive drugs on cytotoxic killing capacity as previously reported^{44, 45}. Cytotoxicity in the "VITAL" assay used for determination of killing capacity is mainly based on perforin/granzyme-mediated killing³⁵. The transcription of perforin in T-cells is reported to be induced by IL-2 and IL-15, thus not exclusively dependent on calcineurin^{46, 47}. Substantial release of granzymes has also been reported in the presence of Tacrolimus⁴⁸, which is in line with our findings for intracellularly captured GZB and increased GZB-producers among CD4⁺ T-cells upon viral stimulus in the presence of immunosuppressants.

Addition of CNIs decreased IFNγ-producers among CD4⁺ and CD8⁺ T-cells, yet IFN□□ production was not completely abolished. This has been observed by others for CD4⁺ memory T-cells at higher doses of IS than used here²³. Indeed, it was reported that IFNγ can also be induced by a calcineurin-independent pathway in T-cells^{49, 50}. This suggests continued IFN□□-production even in presence of CNIs. Additionally, some downstream signaling events of the TCR are unaffected by inhibition of calcineurin and may lead to IFNγ induction⁵¹. However, Tacrolimus significantly reduced the proportion of IFNγ -producers. Cytokine production in the presence of Tacrolimus was rescued after FKBP12^{k.o.}, even in presence of triple IS, although MPA and corticosteroids are reported to further downregulate TCR-mediated IFNγ-production^{21, 24}. In contrast, TNFα-producers were much more sensitive to inhibition by calcineurin inhibitors. Nonetheless, FKBP12^{k.o.}-CMV-TCPs recovered their ability of TNFα production in the presence of Tacrolimus, confirming reports identifying calcineurin as the crucial inducer of TNF□⁵². As previously reported⁵³, we found IL-2-production by T-cells to be highly sensitive to immunosuppressants. Our data revealed significant recovery of IL-2-producers in FKBP12^{k.o.}-CMV-TCPs in the presence of Tacrolimus. Notably, IL-2 is primarily produced by less differentiated long-lived memory T-cells with high proliferative potential, which are a very attractive cell subset for antiviral T-cell therapy approaches³⁷. Polyfunctional IFNγ⁺ TNFα⁺ IL-2⁺ CD4⁺ and CD8⁺ anti-viral T-cells were found to be protective against CMV disease after SOT⁵⁴. Our strategy for FKBP12^{k.o.} retained IFNγ/TNFα-double-producers and IL-2-producers despite immunosuppression, hence, the FKBP12^{k}-CMV-TCP is an attractive adoptive T-cell transfer approach for the induction of protective immunity *in vivo*⁵⁴*.* The activation marker CD154, which is associated with polyfunctionality⁵⁵ and protection from CMV after SOT⁴⁰, was also highly sensitive to immunosuppressants. Of note, CMV-specific CD154 upregulation in the presence of Tacrolimus could be restored by FKBP12^{k.o.}, underpinning the high antiviral potential of FKBP12^{k.o.}-CMV-TCPs in presence of Tacrolimus. CD154 is also expressed on a small subset of virus-specific CD8⁺ T-cells (here <10%), which may exert helper-like functions³⁹. We did not observe any effect of CNIs on the CMV-stimuli-induced upregulation of the activation marker CD137 (4-1BB) on the T-cells examined. Glucocorticoid treatment is reported to transiently increase proportions of CD25⁺CD4⁺ T-cells⁵⁶. Indeed, following 24 h of incubation with triple IS including prednisolone, we recorded a transiently elevated expression of CD25 that progressively declined by day 4 of culture in triple IS. MPA was reported to downregulate CD25 expression on T-cells⁵⁷. This possibly counteracted the glucocorticoid-mediated upregulation, resulting in the decline observed by day 4 of culture in triple IS. We could not confirm data implying a glucocorticoid- or MPA-mediated upregulation of PD-1 on T-cells^{21, 58}. MPA and dexamethasone have been suggested to upregulate expression of CTLA-4 on CD4⁺ T-cells^{21, 59}. However, we observed significant attenuation of CTLA-4 after 4 days of culture in triple IS. CTLA-4 expression requires antigen stimulation⁶⁰. We presume the single initial antigenic T-cell stimulation at day 0 was insufficient to sustain CTLA-4 expression.

Gene editing may raise concerns about transformation of the edited cells, which could lead to uncontrolled proliferation and eventually tumor formation. An increase in clonality of the TCPs could be an indicator of transformation, since certain transformed clones gain a growth advantage and are able to outgrow other clones. Thus, we performed TCR sequencing to exclude that there were any transformative events in the TCPs. We found that the FKBP12^{k.o.}-CMV-TCPs did not have higher clonality than their non-edited counterparts, implying the cells were not transformed. Furthermore, the percentage covered by the top 10 most frequent clones was also not increased, confirming that none of the clones had acquired proliferative advantages as a result of gene editing.

Use of microbial compounds for genetic engineering and introduction of genetically modified cells into humans may elicit immune reactions⁶¹. We have demonstrated that FKBP12^{k.o.}-CMV-specific T-cells degrade the Cas9 protein within 5 days post-electroporation. Thus, recombinant bacterial Cas9, which was reported to elicit adaptive immune responses^{41, 42}, is unlikely to cause immune reactions in patients treated with the TCP described here. In general, the use of autologous cells bears a low risk of immunogenicity¹⁹. Nonetheless, potential mutations inserted during the repair of double strand breaks may result in immunogenicity of the edited cells and cause T-cell activation. The major concern when applying CRISPR-Cas9 technology for genetic engineering remains off-target gene-editing posing the risk of uncontrollable mutagenesis⁶². In depth characterization applying next-generation-sequencing technology to investigate potential off-target editing events⁶³ predicted by the COSMID tool *in silico*³¹ and using *in vitro* assays^{64, 65} is performed to ensure a complete risk-safety calculation for clinical application⁶⁶.

Within the initially sorted CMV-specific CD8⁺ T-cells, there was a high percentage of terminally differentiation T_{EMRA}. This could raise concerns about the efficacy, longevity and induction of functional memory by the resulting TCP. However, T_{EMRA} are strongly associated with CMV infection, which induces them at high levels⁶⁷. They were shown not to expand in polyclonally activated CAR-TCPs, when cultured either alone or in bulk cultures of different T-cell memory subsets⁶⁸. This is in line with our findings for the expanded and thawed TCPs, which are likely to result from proliferation of less differentiated memory T-cell subsets.

In summary, the invention provides a GMP-compatible protocol for the manufacture of calcineurin inhibitor-resistant T cells, e.g., Tacrolimus-resistant CMV-specific TCPs, which are functionally superior to unmodified antiviral TCPs in the presence of calcineurin inhibitor, e.g., Tacrolimus, and triple IS. Furthermore, they contain features of highly potent protective early memory T-cells as described herein. In case of an FKBP12^{k.o.,} the sensitivity to CsA represents an inherent safety switch, which is exploitable in case the product should elicit adverse effects. Additionally, compared to previous approaches to generate CNI-resistant TCPs²⁶⁻²⁸ our product is vector free and utilizes targeted mutagenesis. As such, it provides a safer methodology for TCP generation, and a safer T cell product.

This manufacturing process can be applied to generate TCPs directed against other viruses or tumors using the respective peptide pools for the initial sorting of T-cells with the desired specificity. The invention thus provides novel pharmaceutical compositions for use in human patients comprising the cells of the invention, e.g., the Tacrolimus-resistant anti-viral TCPs of the invention.

### Materials and Methods

### Blood sampling, isolation and culture of virus-specific T-cells

The study was approved by the Charité-Universitätsmedizin Berlin Ethics Committee and peripheral blood was obtained from healthy donors, who had given their written informed consent. PBMCs were isolated using Biocoll (Biochrom) gradient centrifugation. Virus-specific T-cells were isolated following a 6 h stimulation with overlapping CMV-specific phosphoprotein (pp)65 and immediate early (IE)-1 peptide pools (JPT Peptide Technologies; 0.5 µg/ml each) using an IFNγ Secretion Assay-Cell Enrichment and Detection Kit according to the manufacturer's instructions (Miltenyi Biotec) and cultured in complete media (VLE RPMI 1640 supplemented with penicillin (100 IU/ml) and streptomycin [all from Biochrom] and 10% fetal calf serum [FCS, PAA]), supplemented with 10 ng/ml recombinant human (rh)IL-7 and rhIL-15 (CellGenix) on 96- or 24-well plates, respectively, in humidified incubators at 37 °C and 5 % CO₂ as described previously^{13, 14}. Cells were split 1:1 upon reaching 100 % confluency. After 21 days, TCPs were frozen in FCS with 10 % dimethyl sulfoxide (Sigma-Aldrich) at - 80°C and transferred to liquid nitrogen after 1 day. TCPs were thawed in a water bath at 37 °C and washed twice with complete medium. One half was analyzed immediately and the other half was seeded into 24-well plates and cultured in complete medium with or without immunosuppressive drugs at clinically relevant doses (6 ng/ml Tacrolimus [Prograf, Astellas]; 120 ng/ml CsA [Sandimmun, Novartis]; triple IS = 6 ng/ml Tacrolimus + 0.57 µg/ml Prednisolone [Urbason solubile, Sanofi] + 2.7 µg/ml mycophenolic acid [MPA, active substance of mycophenolate mofetil, Sigma-Aldrich]) until further analysis.

### Knockout procedure

FKBP12-specific sgRNAs (Table 1) were *in vitro* transcribed from DNA templates as previously described⁶⁹, using Hi-Scribe T7 High Yield RNA Synthesis Kit (New England Biosciences). Where indicated, synthetic modified sgRNA (Synthego Corporation) with 2O'-methyl-3'phosphothioate modifications between the first and last 3 nucleotides was used.

2.5 million antiviral T-cells were electroporated at day 7 after isolation using Amaxa P3 primary cell 4D-Nucleofector X Kit L and the Amaxa-Nucleofector-4D (Lonza, program CO-115) to transfer ribonucleoprotein complexes of 30 µg of recombinant Alt-R *Streptococcus pyogenes* Cas9 protein V3 (Integrated DNA Technologies) precomplexed with 30 µg of *in-vitro*-transcribed or 15 synthetic sgRNA (Synthego Corporation). The same number of unmodified antiviral T-cells and antiviral T-cells electroporated without additives were expanded as controls.

### Phenotypic and functional assays assessed by flow cytometry

For assessment of CMV-specific cytokine production/activation, lymphoblastic B-cell lines (LCLs) were generated as described previously⁷⁰ and used as antigen presenting cells at a 1:10 ratio for a 6 h CMV-specific stimulation with 0.5 µg/ml CMV-specific peptide pools (IE-1 and pp-65 each) in the presence or absence of immunosuppressants at clinical doses indicated above. Unstimulated controls included LCLs without CMV-peptides. Intracellular cytokine production was captured by addition of 2 µg/ml of Brefeldin A (Sigma-Aldrich) after 1 h of stimulation and cells were stained using antibodies (all from BioLegend, unless stated otherwise) and the FoxP3/Transcription Factor Staining Buffer Set (eBioscience). Staining was performed using fluorophore-conjugated human anti-CD3 (OKT3), - CD4 (SK3), -CD8 (RPA-T8), -IFNγ (4S.B3, eBioscience), -TNFα (MAb11), -IL-2 (MQ1-17H12), - CD137 (4B4-1), -CD154 (24-31) and -Granzyme B (GZB; GB11; BD Pharmingen) antibodies. LIVE/DEAD Fixable Blue Dead Cell Stain (L/D; Invitrogen) was used to identify living cells, which was also used for determination of viability in combination with fluorescently labelled Annexin V (BioLegend).

A "VITAL" assay was performed to assess killing capacity of TCPs ^{14, 35, 36}. Briefly, cells from TCPs were incubated at distinct ratios with CMV-peptide-loaded autologous LCLs stained with 10 µM Car-boxyfluorescein Diacetate Succinimidyl Ester (CFSE-DA; Sigma-Aldrich) for 4 min and unloaded allogenic LCLs stained with 5 µM CellTrace^{™} Far Red (Invitrogen) for 10 min. T-cell-free LCL mixtures served as internal controls to calculate the CMV-specific killing capacity³⁵. After 14 h of incubation, co-cultures were stained with L/D.

All flow cytometry samples were analyzed using an LSR-II-Fortessa flow cytometer (Becton Dickinson) and FlowJo-10 software (Tree Star).

### Efficiency analysis

Analysis of on-target editing was performed from isolated DNA (Zymo Research) of day 21 cell samples. The FKBP12 locus was amplified using KAPA HiFi HotStart ReadyMix (Roche) and the following primer pairs: TCTGACGGGTCAGATAACACCTAG (F, SEQ ID NO: 8) and TCTTCCG-GAGGCCTGGGTTT (R, SEQ ID NO: 9) for sgRNA#1&2; ACAGCTGTATCCGGAGGCCT (F', SEQ ID NO: 10) and TCACAGCCGCCGATTCAGAC (R', SEQ ID NO: 11) for sgRNA#3 (Eurofins Scientific SE) with the following touchdown-PCR program in an automated thermocycler: 1. 95 °C, 3 minutes, 2. 98 °C, 30 seconds 3. 72-64 °C for sgRNA#1&2/68-64 °C for sgRNA#3, 15 seconds (-0.5 °C for each cycle starting at the highest until the lowest temperature was reached; 20 cycles, 64 °C); 4. 72 °C, 15 seconds; 5. repeat from step 2 with decreasing annealing temperature (as specified); 6. 72 °C, 1 minute; 7. 4 °C. PCR products were purified using DNA purification & enrichment kit (Zymo Research) prior Sanger sequencing with primer F/F' by LGC Genomics GmbH. Editing frequencies were calculated using the Inference of CRISPR Edits (ICE) algorithm (Synthego Corporation)63.

### T-cell receptor-β sequencing

T-cell receptor-β (TCRβ) sequencing was performed from genomic DNA by Adaptive Biotechnologies and analyzed using ImmunoSEQ ANALYZER 3.0 (Adaptive Biotechnologies). The data are available at the immuneACCESS platform.

### Example 2 - cyclophilin A^{k.o.} T cells

Peripheral blood mononuclear cells were freshly isolated from healthy human donors and stimulated with plate-bound anti-CD3/anti-CD28 antibodies in the presence of cytokine containing medium (RPMI 1640 medium [Biochrom], 10% heat-inactivated fetal calf serum [Biochrom], 10 ng/ml IL-7 [Cellgenix], 5 ng/ml IL-15 [Cellgenix]). Two days after stimulation, activated T cells were harvested and electroporated with sgRNA-Cas9 complexes using the Neon Electroporation System (Invitrogen). PPIA specific sgRNAs of SEQ ID NO: 4-7 and control CD45 targeting sgRNA as previously described were used (Gundry et al. 2016, Cell Reports). Prior to electroporation, sgRNA were incubated with recombinant Cas9 protein (PNAbio) for 15 minutes at room temperature and electroporation was performed as previously described (Gundry et al. 2016 Cell Reports). Four days after electroporation, genomic DNA was extracted of 200.000 T cells. The PPIA locus was amplified using polymerase chain reaction (Forward primer: 5'- ACTCGCGGACCTCCCAAAATG-3' (SEQ ID NO: 12); Reverse primer: 5'- ACGCGCCTCATCGCTTGACA-3'(SEQ ID NO: 13)). Seven days after electroporation, T cells were restimulated with PMA/Ionomycin for 6 hours in medium alone or in medium supplemented with either Cyclosporine A (therapeutic dose, 120 ng/ml [Sandimmun, Novartis]) or Tacrolimus (therapeutic dose, 6 ng/ml; [Prograf, Astellas]). One hour after begin of stimulation, Brefeldin A was added to prevent cytokine secretion and allow for intracellular staining of cytokines with fluorochrome-labeled antibodies. Subsequent flow cytometry analysis allowed evaluation of cytokine production in alive T cells of different subsets (e.g. CD4+ and CD8+).

### Example 3 - FKBP12^{k.o.} regulatory T cells

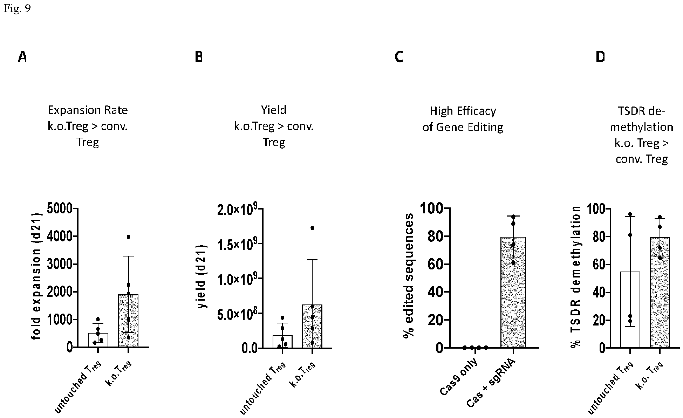

Exemplary regulatory T cells were isolated from peripheral blood mononuclear cells of healthy human adults based on the expression of CD4+ CD25high and low expression of CD127. Isolated Treg were then subsequently stimulated with anti-CD3/anti-CD28 coated expansion beads in the presence of IL-2 (500 IU/ml) and rapamycin. Seven days after inital activation and expansion, CD4+ Treg cells were electroporated with CRISPR-Cas9 ribonucleinprotein complexes comprising of synthetic sgRNA FKBP12 (SEQ ID NO: 1) and recombinant Cas9 protein. After expansion for 21 days, the cells were analysed and compared with untouched Tregs regarding expansion rate and final yield (Fig. 9 A+B), gene editing efficacy measured by Sanger sequencing and ICE analysis (Synthego) (Fig. 9 C) and epigenetic identity, through quantification of demethylation within the Treg-specific demethylation region (TSDR) (Fig. 9 D). FKBP12-KO could be performed with high efficiency and even increased overall yield, presumably due to reduced inhibition by rapamycin treatment. FKBP12-KO Treg retained epigenetic identity of natural Treg as indicated by high TSDR demethylation.

Fig. 10 shows exemplary regulatory T cells, expanded and treated as described in Fig. 9. Edited and control Treg cells were subsequently expanded in medium with or without different calcineurin inhibitors and subsequently analysed for cell expansion (Fig. 10 A) and with flow cytometry (Fig. 10 B+C). As expected, Tacrolimus treatment reduced the proliferation of untouched Tregs, while FKBP12-KO Tregs retained fully proliferative capacity despite the drug treatment (Fig. 10 A). FKBP12-KO Treg displayed higher expression of the inhibitory receptor CTLA4 (Fig. 10 B). CTLA4 is known to suppress T cells and modulate pro-inflammatory responses by dendritic cells. Further, Foxp3 transcription factor expression assessed by flow cytometry was comparable between wildtype and FKBP12-KO, with a trend toward increased Foxp3 expression of FKBP12KO-Treg in the presence of Tacrolimus indicating potential for increased stability (Fig. 10 XX C)

### References

1. Chen, JH, et al. (2005). The impact of pretransplant cytomegalovirus infection on acute renal allograft rejection. Transplant Proc 37: 4203-4207.
2. Tong, CY, et al. (2002). The association of viral infection and chronic allograft nephropathy with graft dysfunction after renal transplantation. Transplantation 74: 576-578.
3. Grossi, PA, et al. (2012). Infections and organ transplantation: new challenges for prevention and treatment--a colloquium. Transplantation 93: S4-S39.
4. Harvala, H, et al. (2013). High risk of cytomegalovirus infection following solid organ transplantation despite prophylactic therapy. J Med Virol 85: 893-898.
5. Kumar, D, et al. (2009). Cell-mediated immunity to predict cytomegalovirus disease in high-risk solid organ transplant recipients. Am J Transplant 9: 1214-1222.
6. Schachtner, T, et al. (2017). CMV-Specific T Cell Monitoring Offers Superior Risk Stratification of CMV-Seronegative Kidney Transplant Recipients of a CMV-Seropositive Donor. Transplantation 101: e315-e325.
7. Hill, GR, , et al. (2010). Successful immunotherapy of HCMV disease using virus-specific T cells expanded from an allogeneic stem cell transplant recipient. Am J Transplant 10: 173-179.
8. Clancy, LE, et al. (2013). Cytomegalovirus-specific cytotoxic T lymphocytes can be efficiently expanded from granulocyte colony-stimulating factor-mobilized hemopoietic progenitor cell products ex vivo and safely transferred to stem cell transplantation recipients to facilitate immune reconstitution. Biol Blood Marrow Transplant 19: 725-734.
9. Stemberger, C, et al. (2014). Lowest numbers of primary CD8(+) T cells can reconstitute protective immunity upon adoptive immunotherapy. Blood 124: 628-637.
10. Stuehler, C, et al. (2015). Combination therapy for multidrug-resistant cytomegalovirus disease. Transpl Infect Dis 17: 751-755.
11. Neuenhahn, M, et al. (2017). Transfer of minimally manipulated CMV-specific T cells from stem cell or third-party donors to treat CMV infection after allo-HSCT. Leukemia.
12. Riddell, SR, et al. (1992). Restoration of viral immunity in immunodeficient humans by the adoptive transfer of T cell clones. Science 257: 238-241.
13. Brestrich, G, et al. (2009). Generation of HCMV-specific T-cell lines from seropositive solid-organ-transplant recipients for adoptive T-cell therapy. J Immunother 32: 932-940.
14. Amini, L, et al. (2019). Comprehensive Characterization of a Next-Generation Antiviral T-Cell Product and Feasibility for Application in Immunosuppressed Transplant Patients. Frontiers in Immunology 10.
15. Macesic, N, et al. (2015). Adoptive T cell immunotherapy for treatment of ganciclovir-resistant cytomegalovirus disease in a renal transplant recipient. Am J Transplant 15: 827-832.
16. Holmes-Liew, CL, et al. (2015). Adoptive T-cell immunotherapy for ganciclovir-resistant CMV disease after lung transplantation. Clin Transl Immunology 4: e35.
17. Smith, C, et al. (2018). Autologous adoptive T-cell therapy for recurrent or drug-resistant cytomegalovirus complications in solid organ transplant patients: A single-arm open-label phase I clinical trial. Clin Infect Dis.
18. Einsele, H, et al. (2002). Infusion of cytomegalovirus (CMV)-specific T cells for the treatment of CMV infection not responding to antiviral chemotherapy. Blood 99: 3916-3922.
19. Brestrich, G, et al. (2009). Adoptive T-cell therapy of a lung transplanted patient with severe CMV disease and resistance to antiviral therapy. Am J Transplant 9: 1679-1684.
20. Savoldo, B, et al. (2006). Treatment of solid organ transplant recipients with autologous Epstein Barr virus-specific cytotoxic T lymphocytes (CTLs). Blood 108: 2942-2949.
21. He, X, et al. (2011). Mycophenolic acid-mediated suppression of human CD4+ T cells: more than mere guanine nucleotide deprivation. Am J Transplant 11: 439-449.
22. Allison, AC, and Eugui, EM (2000). Mycophenolate mofetil and its mechanisms of action. Immunopharmacology 47: 85-118.
23. Tsuda, K, et al. (2012). Calcineurin inhibitors suppress cytokine production from memory T cells and differentiation of naive T cells into cytokine-producing mature T cells. PLoS One 7: e31465.
24. Brinkmann, V, and Kristofic, C (1995). Regulation by corticosteroids of Th1 and Th2 cytokine production in human CD4+ effector T cells generated from CD45RO- and CD45RO+ subsets. J Immunol 155: 3322-3328.
25. Herold, MJ, et al. (2006). Glucocorticoids in T cell apoptosis and function. Cellular and molecular life sciences : CMLS 63: 60-72.
26. Brewin, J, et al. (2009). Generation of EBV-specific cytotoxic T cells that are resistant to calcineurin inhibitors for the treatment of posttransplantation lymphoproliferative disease. Blood 114: 4792-4803.
27. Ricciardelli, I, et al. (2013). Rapid generation of EBV-specific cytotoxic T lymphocytes resistant to calcineurin inhibitors for adoptive immunotherapy. Am J Transplant 13: 3244-3252.
28. De Angelis, B et al. (2009). Generation of Epstein-Barr virus-specific cytotoxic T lymphocytes resistant to the immunosuppressive drug tacrolimus (FK506). Blood 114: 4784-4791.
29. Xu, X, et al. (2002). FKBP12 is the only FK506 binding protein mediating T-cell inhibition by the immunosuppressant FK506. Transplantation 73: 1835-1838.
30. Gundry, MC, et al. (2016). Highly Efficient Genome Editing of Murine and Human Hematopoietic Progenitor Cells by CRISPR/Cas9. Cell Rep 17: 1453-1461.
31. Cradick, TJ, et al. (2014). COSMID: A Web-based Tool for Identifying and Validating CRISPR/Cas Off-target Sites. Molecular Therapy - Nucleic Acids 3: e214.
32. Casper, J, Zweig, AS, Villarreal, C, Tyner, C, Speir, ML, Rosenbloom, KR, et al. (2018). The UCSC Genome Browser database: 2018 update. Nucleic Acids Research 46: D762-D769.
33. Hendel, A, et al. (2015). Chemically modified sgRNAs enhance CRISPR-Cas genome editing in human primary cells. Nature Biotechnology 33: 985.
34. Kannanganat, S, et al. (2007). Multiple-cytokine-producing antiviral CD4 T cells are functionally superior to single-cytokine-producing cells. J Virol 81: 8468-8476.
35. Hammoud, B, et al. (2013). HCMV-specific T-cell therapy: do not forget supply of help. J Immunother 36: 93-101.
36. Hermans, IF, et al. (2004). The VITAL assay: a versatile fluorometric technique for assessing CTL- and NKT-mediated cytotoxicity against multiple targets in vitro and in vivo. J Immunol Methods 285: 25-40.
37. Sallusto, F, et al. (2004). Central memory and effector memory T cell subsets: function, generation, and maintenance. Annu Rev Immunol 22: 745-763.
38. Arce, S, et al. (2007). In vitro induction of immunoglobulin A (IgA)- and IgM-secreting plasma blasts by cholera toxin depends on T-cell help and is mediated by CD 154 upregulation and inhibition of gamma interferon synthesis. Infect Immun 75: 1413-1423.
39. Frentsch, M, et al. (2013). CD40L expression permits CD8+ T cells to execute immunologic helper functions. Blood 122: 405-412.
40. Ashokkumar, C, et al. (2020). CD 154-expressing CMV-specific T cells associate with freedom from DNAemia and may be protective in seronegative recipients after liver or intestine transplantation. Pediatr Transplant 24: e13601.
41. Wagner, DL, et al. (2018). High prevalence of Streptococcus pyogenes Cas9-reactive T cells within the adult human population. Nat Med.
42. Charlesworth, CT, et al. (2019). Identification of preexisting adaptive immunity to Cas9 proteins in humans. Nat Med 25: 249-254.
43. Kaeuferle, T, et al. (2020). CRISPR-Cas9-Mediated Glucocorticoid Resistance in Virus-Specific T Cells for Adoptive T Cell Therapy Posttransplantation. Mol Ther.
44. Strauss, G, et al. (2002). Induction of apoptosis and modulation of activation and effector function in T cells by immunosuppressive drugs. Clin Exp Immunol 128: 255-266.
45. Savoldo, B, et al. (2001). Generation of autologous Epstein-Barr virus-specific cytotoxic T cells for adoptive immunotherapy in solid organ transplant recipients. Transplantation 72: 1078-1086.
46. Zhang, J, et al. (1999). Interleukin 2 receptor signaling regulates the perforin gene through signal transducer and activator of transcription (Stat)5 activation of two enhancers. J Exp Med 190: 1297-1308.
47. Alves, NL, H et al. (2003). IL-15 induces antigen-independent expansion and differentiation of human naive CD8+ T cells in vitro. Blood 102: 2541-2546.
48. Kataoka, T (2000). Involvement of FK506-sensitive and insensitive granule exocytosis pathways in perforin-dependent target cell lysis mediated by a CD8+ CTL clone. Immunology Letters 72: 49-52.
49. Yang, J, et al. (1999). Induction of interferon-y production in Th1 CD4+ T cells: evidence for two distinct pathways for promoter activation. European Journal of Immunology 29: 548-555.
50. Balasubramani, A, et al. (2010). Modular utilization of distal cis-regulatory elements controls Ifng gene expression in T cells activated by distinct stimuli. Immunity 33: 35-47.
51. Dutta, D, et al. (2017). Recruitment of calcineurin to the TCR positively regulates T cell activation. Nat Immunol 18: 196-204.
52. Goldfeld, AE, et al. (1994). Calcineurin mediates human tumor necrosis factor alpha gene induction in stimulated T and B cells. J Exp Med 180: 763-768.
53. O'Keefe, SJ, et al. (1992). FK-506- and CsA-sensitive activation of the interleukin-2 promoter by calcineurin. Nature 357: 692-694.
54. Snyder, LD, et al. (2016). Polyfunctional T-Cell Signatures to Predict Protection from Cytomegalovirus after Lung Transplantation. Am J Respir Crit Care Med 193: 78-85.
55. (2004). Cytomegalovirus. Am J Transplant 4 Suppl 10: 51-58.
56. Braitch, M, et al. (2009). Glucocorticoids increase CD4CD25 cell percentage and Foxp3 expression in patients with multiple sclerosis. Acta Neurol Scand 119: 239-245.
57. Premaud, A, et al. (2011). Inhibition of T-cell activation and proliferation by mycophenolic acid in patients awaiting liver transplantation: PK/PD relationships. Pharmacol Res 63: 432-438.
58. Maeda, N, et al. (2019). Glucocorticoids potentiate the inhibitory capacity of programmed cell death 1 by up-regulating its expression on T cells. J Biol Chem 294: 19896-19906.
59. Xia, M, et al. (1999). Dexamethasone enhances CTLA-4 expression during T cell activation. Cell Mol Life Sci 55: 1649-1656.
60. Gibson, HM, et al. (2007). Induction of the CTLA-4 gene in human lymphocytes is dependent on NFAT binding the proximal promoter. J Immunol 179: 3831-3840.
61. Riddell, SR, et al. (1996). T-cell mediated rejection of gene-modified HIV-specific cytotoxic T lymphocytes in HIV-infected patients. Nature Medicine 2: 216.
62. Fu, Y, et al. (2013). High-frequency off-target mutagenesis induced by CRISPR-Cas nucleases in human cells. Nature Biotechnology 31: 822.
63. Hsiau, T, et al. (2019). Inference of CRISPR Edits from Sanger Trace Data. BioRxiv.
64. Turchiano, G, et al. (2020). Quantitative Evaluation of Chromosomal Rearrangements in Primary Gene-Edited Human Stem Cells by Preclinical CAST-Seq. SSRN Electronic Journal.
65. Cameron, P, et al. (2017). Mapping the genomic landscape of CRISPR-Cas9 cleavage. Nat Methods 14: 600-606.
66. Cathomen, T, et al. (2019). The Human Genome Editing Race: Loosening Regulatory Standards for Commercial Advantage? Trends Biotechnol 37: 120-123.
67. Di Benedetto, S, et al. (2015). Impact of age, sex and CMV-infection on peripheral T cell phenotypes: results from the Berlin BASE-II Study. Biogerontology 16: 631-643.
68. Schmueck-Henneresse, M, et al. (2017). Comprehensive Approach for Identifying the T Cell Subset Origin of CD3 and CD28 Antibody-Activated Chimeric Antigen Receptor-Modified T Cells. J Immunol 199: 348-362.
69. Li, D, et al. (2013). Heritable gene targeting in the mouse and rat using a CRISPR-Cas system. Nat Biotechnol 31: 681-683.
70. Moosmann, A, et al. (2002). B cells immortalized by a mini-Epstein-Barr virus encoding a foreign antigen efficiently reactivate specific cytotoxic T cells. Blood 100: 1755-1764.
71. Basar R., et al. (2020). Large-scale GMP-compliant CRISPR-Cas9-mediated deletion of the glucocorticoid receptor in multivirus-specific T cells. Blood Advances 4(14): 3357-3367.
72. Houghtelin, A and Bollard, CM (2017). Virus-specific T cells for the immunocompromised patient. Frontiers in Immunology 8, Art. 1272.
73. Hendel et al. 2015. Nat Biotechnology 33(9):985-989.
74. Gundry et al. 2016. Cell Reports 17(5): 1453-1461.
75. WO2014/191128 A1
76. WO 2012/171882 A1

## Claims

1. A human immunophilin knockout cell that is a T cell or NK cell, wherein the immunophilin is FKBP12 or Cyclophilin A.

2. The cell of claim 1, wherein the immunophilin is FKBP-12.

3. The cell of claim 1, wherein the immunophilin is Cyclophilin A.

4. The cell of any of the preceding claims, wherein the cell is a T cell selected from the group comprising a T cell specific for a virus, another pathogen or a cancer antigen,
preferably, a virus-specific T cell, wherein the virus is selected from the group comprising CMV, EBV, BKV, HPV, ADV, influenza virus, parvovirus, rubella virus, hepatitis viruses (HBV, HCV, HAV, HDV, HEV), coxsackie virus, respiratory syncytial virus (RSV), coronaviruses, such as but not only: MERS, SARS-CoV1 and SARS-CoV-2.

5. The cell of any of the preceding claims, wherein the cell is a regulatory T cell, preferably, a CD4+CD25+ regulatory T cell, wherein, optionally, the antigen is a self-antigen or an allogeneic antigen.

6. The cell of any of claims 1-3, wherein the cell is an NKT cell, an NK cell or a γδ T cell, preferably, an NKT cell.

7. The cell of any of the preceding claims, wherein the cell is obtainable by CRISPR/Cas-mediated gene editing of the immunophilin gene.

8. A population of cells comprising cells of any of claims 1-7, wherein the population is a polyclonal population of cells,
wherein, preferably, the population comprises cells with InDel mutations in the genes encoding the immunophilin,
wherein, optionally, the cell population is obtainable from gene editing of the immunophilin genes of a respective cell population obtained from a human subject, e.g., by Cas-mediated gene editing.

9. A method for preparing a human immunophilin knockout T cell or NK cell of any of claims 1-7 or a T cell or NK cell population of claim 8, comprising
a) stimulating T cells or NK cells obtained from a subject,
b) isolating stimulated T cells or NK cells based on expression of a marker to obtain a composition of selected T cells or NK cells,
c) gene editing the cells of said composition to knock out the gene encoding the immunophilin, preferably, introducing a ribonucleoprotein complex comprising a CRISPR associated protein (Cas) and a guide RNA targeting the gene encoding the immunophilin into the cells of said composition, and
d) optionally, selecting immunophilin knockout cells by culturing the cells in the presence of an immunosuppressant agent capable of interacting with the immunophilin,
wherein, preferably, the cells are T cells.

10. The method of claim 9, wherein the immunophilin is FKBP12 and the single sgRNA targets the sequence of SEQ ID NO: 1, wherein the sgRNA preferably is a synthetic sgRNA.

11. The method of claim 9, wherein the immunophilin is cyclophilin A and the sgRNA targets the sequence of SEQ ID NO: 5 or 6, wherein the sgRNA preferably is a synthetic sgRNA.

12. The method of any of claims 9-11, wherein the human immunophilin knockout cell is a T cell specific for a virus, another pathogen or a cancer antigen, wherein, in step a), the T cells are stimulated with a virus-derived antigen, another pathogen-derived antigen or a cancer antigen, and in step b), the marker is an activation marker selected from the group comprising IFNy-secretion.

13. The method of any of claims 9-12, wherein the human immunophilin knockout cell is a regulatory T cell, wherein preferably, in step b), the marker is a regulatory T cell marker selected from the group comprising CD25.

14. The cell of any of claims 1-7 or the cell population of claim 8, wherein the cell is obtainable by carrying out the method of any of claims 9-13.

15. A pharmaceutical composition comprising the cell of any of claims 1-7 or the cell population of claim 8, or
a kit comprising said pharmaceutical composition and an immunosuppressant agent capable of interacting with the immunophilin, wherein the kit optionally further comprises an immunosuppressant agent capable of inhibiting an immune response of the cells.

16. The pharmaceutical composition of claim 15, wherein the T cell(s) is a T cell specific for a virus, another pathogen or a cancer antigen, or an NK cell, for use in treatment or prevention of an infection with the virus or the other pathogen or for use in treatment of the cancer,
wherein, preferably, the treated subject is immunosuppressed with an immunosuppressant agent capable of interacting with the immunophilin.

17. The pharmaceutical composition of claim 15, wherein the cell is a regulatory T cell, for use in balancing an unwanted immune response in a patient having a condition selected from the group comprising autoimmunity, autoinflammation, allergy, a transplantation and an immunopathology caused by bystander activation.

18. An immunosuppressive agent capable of interacting with an immunophilin selected from the group consisting of Tacrolimus and cyclosporine A for use suppressing an undesired immune response in a patient who is administered a pharmaceutical composition of any of claims 15-17,
wherein, preferably, said patient could not otherwise have been treated with the immunosuppressant to avoid lack of a desired immune response selected from an immune response to a virus, another pathogen or a cancer, wherein the undesired immune response optionally is bystander activation of T cells, e.g., caused by a virus selected from the group comprising SARS-CoV-2.
